# EUROPEAN PATENT APPLICATION

(11) **EP 1 526 131 A1**
(43) Date of publication of application: **27.04.2005**
(21) Application number: 03024255.6
(22) Date of filing: 22.10.2003
(51) Int. Cl.: C07D 241/20, C07D 409/14, C07D 401/12, C07D 409/12, C07D 401/14, A61K 31/497, A61P 7/02

(54) **Aminoalkyl-pyrazinones and -pyridones as thrombin inhibitors**

(71) Applicant: Graffinity Pharmaceuticals Aktiengesellschaft, 69120 Heidelberg (DE)
(72) Inventor: Feurer, Achim, 69256 Wilhelmsfeld (DE); Bosio, Sara, 68165 Mannheim (DE); Bulat, Stephan, 69118 Ziegelhausen (DE); Cerezo-Galvez, Silvia, 69118 Heidelberg (DE); Matassa, Victor Giulio, 69493 Hirschberg (DE); Ott, Inge, 68775 Ketsch (DE); Papadopoulos, Michael Arthur, 69124 Heidelberg (DE); Rozenbaum, Claudia, 50354 Hürth (DE); Schamberger, Jens, 69123Wieblingen (DE); Metz, Günther, 69126 Heidelberg (DE)
(74) Representative: Grünecker, Kinkeldey, Stockmair & Schwanhäusser

(57) **Abstract**

The invention relates to compounds of formula (I) 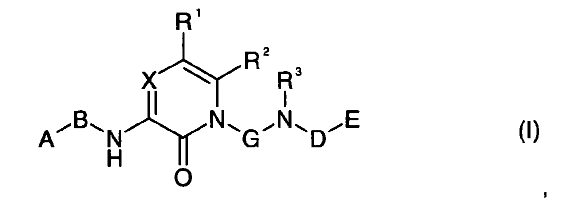 wherein A, B, X, R¹, R², G, R³, D and E have the meaning as cited in the description and the claims. Said compounds are useful as coagulants. The invention also relates to the production and use thereof as medicament.

## Description

The present invention relates to aminoalkyl-pyrazinones and -pyridones having an antithrombotic effect and their prodrugs useful as anticoagulants for the treatment or prophylaxis of thrombin related diseases.

Venous and arterial thromboembolism may cause pulmonary embolism, myocardial infarction and ischaemic stroke and hence are a major cause for morbidity and mortality. Therefore, significant efforts have been made to find effective antithrombotic therapies. The list of established drugs for the prevention of thrombus formation and embolisation include low molecular weight heparins, hirudin and derivatives, aspirin, thienopyridine-type ADP- receptor antagonists and glycoprotein IIb/IIIa receptor antagonists, as well as vitamin K antagonists. Several limitations caused some these therapies being of only limited use or leading to severe implications. These limitations in current therapies have stimulated the search for new and more efficient anticoagulants.

Thrombin is a serine protease present in blood plasma in the form of its precursor, prothrombin (Mann, K.G., *Biochemistry and physiology of blood coagulation, Thromb. Haemost.* 1999, 82, 165-74) and plays a central role in the mechanism of blood coagulation by converting the soluble plasma protein fibrinogen into the insoluble fibrin which forms a clot. In addition, thrombin transforms coagulation factor XII to factor VIIIa which covalently cross-links the fibrin strands. Thrombin is responsible for a variety of cellular actions mediated by binding to specific protease-activated receptors (O'Brien, P.J. et al. *Protease activated receptors: theme and variations. Oncogene* 2001, 20, 1570-81). In addition, thrombin is one of the most potent stimulators of platelet aggregation and also a potent mitogen for vascular muscle cells.

Due to its multiple physiological actions in the context of blood coagulation, thrombin provides evidence to be a suitable target for drug discovery and development.

3-Amino-2-pyridone and 5-amino-6-pyrimidone acetamide templates are described as effective surrogates for the glycylproline dipeptide backbone of inhibitors of human leukocyte elastase (Brown, F.J., et al., J. Med.Chem, 1994, 37, 1259-61).
In US5668289 (1997), WO9831670 (1998), WO9730708 (1997) and WO9701338 (1997) several sulfonylated pyridone acetamides are described to be potent and selective inhibitors of thrombin. Further pyridone acetamides are described in WO0032574 (2000) and WO9926926 (1999).
In WO9740024 (1997) Pyrazinone acetamides are described to be potent inhibitors of thrombin. Structural variations led to further pyrazinone acetamides published in WO9911267 (1999), WO9961442 (1999), WO9959591 (1999), WO0026210 (2000). EP-0997474 discloses further pyrazinone acetamides as thrombin inhibitors. A further series of Pyridones and Pyrazinones described to show activity as thrombin inhibitors is comprised by US 2003/0092679.

However, the compounds described so far can't suffice the demanding needs for effective antithrombotic agents, anticoagulants or thrombin inhibitors.

Thus, the object of the present invention is to provide novel and selective compounds which can overcome at least some of the draw backs of the compounds from the state of the art.

Accordingly, the present invention provides compounds of formula (I): 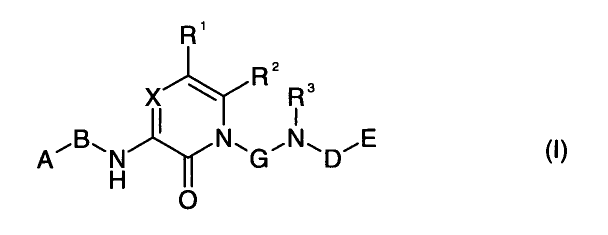 or pharmaceutically acceptable salts thereof, wherein:
- R¹: is hydrogen;
halogen; or
C₁₋₄ alkyl, optionally substituted with one or more fluoro;
- R²: is hydrogen;
halogen;
C₁₋₆ alkyl, optionally substituted with one or more fluoro;
C₃₋₆ cycloalkyl; or
O-C₁₋₄ alkyl;
- R³: is hydrogen;
C₁₋₄ alkyl; or
C₃₋₆ cycloalkyl;
- A: is A¹, wherein A¹ is selected from the group consisting of:
phenyl;
naphtyl;
heterocycles containing up to 4 heteroatoms, which are the same or different and
   selected from the group consisting of -O-, -S-, -S(O)-, -S(O₂)-, -N=, -N(O)= and -N(R⁴)-; and
heterobicycles containing up to 6 heteroatoms, which are the same or different and selected from the group consisting of -O-, -S-, -S(O)-, -S(O₂)-, -N=, -N(O)= and -N(R⁴)-;
   wherein A¹ is optionally substituted with one or independently from each other more of
   A²;
   A³;
   halogen;
   -N(R⁵R⁶);
   -OH;
   =O, where the ring is at least partially saturated;
   C₃₋₆ cycloalkyl;
   -COOR⁷; or
   -CONR⁸R⁹;
and wherein R⁴, R⁵, R⁶ are independently selected from the group consisting of hydrogen, C₁₋₄ alkyl and -C(O)-C₁₋₄ alkyl;
and wherein R⁷, R⁸, R⁹, are independently hydrogen or C₁₋₄ alkyl;
- A²: is selected from the group consisting of A⁴, -O-A⁴ and -N(R¹⁰)-A⁴,
wherein A⁴ is phenyl or a heterocycle containing up to 4 heteroatoms, which are the same or different and selected from the group consisting of -O-, -S-, -S(O)-, -S(O₂)-, -N=, -N(O)= and -N(R¹¹)-; wherein A⁴ is optionally substituted with one or independently from each other more of
fluoro;
chloro;
-N(R¹²R¹³)
C₁₋₄ alkyl or -O-C₁₋₄ alkyl, both optionally substituted with one or independently from each other more of fluoro or -N(R¹⁴R¹⁵);
and wherein R¹⁰, R¹², R¹³, R¹⁴, R¹⁵ are independently hydrogen or C₁₋₄ alkyl;
and wherein R¹¹ is selected from the group consisting of hydrogen, C₁₋₄ alkyl and -C(O)-C₁₋₄ alkyl;
- A³: is selected from the group consisting of C₁₋₆ alkyl, -O-C₁₋₆ alkyl and -N(R¹⁶)-C₁₋₆ alkyl, wherein the C₁₋₆ alkyl group is optionally substituted with one or independently from each other more of
fluoro;
-N(R¹⁷R¹⁸);
A⁵;
and/or A³ is optionally interrupted with one or more oxygen;
and wherein R¹⁶, R¹⁷, R¹⁸ are independently hydrogen or C₁₋₄alkyl;
- A⁵: is phenyl or a heterocycle containing up to 4 heteroatoms, which are the same or different and selected from the group consisting of -O-, -S-, -S(O)-, -S(O₂)-, -N=, -N(O)= and -N(R¹⁹)-; wherein A⁵ is optionally substituted with one or independently from each other more of
fluoro;
chloro;
-N(R²⁰R²¹)
C₁₋₄ alkyl or -O-C₁₋₄ alkyl, both optionally substituted with one or independently from each other more of fluoro or -N(R²²R²³);
and wherein R¹⁹ is selected from the group consisting of hydrogen, C₁₋₄ alkyl and -C(O)-C₁₋₄ alkyl;
and wherein R²⁰, R²¹, R²², R²³ are independently hydrogen or C₁₋₄ alkyl;
- B: is selected from the group consisting of -Y-Z-; -Y-Z-C(O)-; -Y-Z-O-C(O)-; -Y-Z-S(O)₂-; and -Y-Z-NH-C(O)- wherein
Y is a bond, -O-, -S-, -N(R²⁴)-, -N(R²⁵)-C(O)-, -C(O)-N(R²⁶)-, or -C(O)-;
Z is C₁₋₆ alkyl,
optionally interrupted with oxygen, sulfur or -N(R²⁷)-
and/or optionally substituted with one or independently from each other more of
halogen;
C₃₋₆ cycloalkyl;
-COOR²⁸;
-CON(R²⁹R³⁰)
and/or optionally one chain carbon forms part of a C₃₋₆ cycloalkyl;
and wherein R²⁴, R²⁵, R²⁶, R²⁷ , R²⁸, R²⁹, R³⁰ are independently
hydrogen; or
C₁₋₄ alkyl, optionally substituted with -COOR³¹ or -CON(R³²R³³)
wherein R³¹, R³², R³³ are independently hydrogen or C₁₋₄ alkyl;
- X: is =C(R³⁴)- or =N-, wherein R³⁴ is
hydrogen;
C₁₋₆ alkyl, optionally substituted with one or more fluoro; or
-S(O)₂R³⁵, wherein R³⁵ is selected from the group consisting of X¹, C₁₋₆ alkyl,
and -C₁₋₆ alkyl-X¹; wherein R³⁵ is optionally substituted with one or
independently from each other more of
fluoro;
chloro;
C₁₋₄ alkyl; or
-O-C₁₋₄ alkyl;
- X¹: is phenyl or a heterocycle containing up to 4 heteroatoms, which are the same or different and selected from the group consisting of -O-, -S-, -S(O)-, -S(O₂)-, -N=, -N(O)= and -N(R³⁶)-; and wherein R³⁶ is selected from the group consisting of hydrogen, C₁₋₄ alkyl and -C(O)-C₁₋₄ alkyl;
- G: is -CH(R³⁷)-C(R³⁸R³⁹)-;
-CH(R³⁷)-C(R³⁸R³⁹)-C(R⁴⁰R⁴¹)-;
wherein R³⁷, R³⁸, R³⁹, R⁴⁰, R⁴¹ are independently
hydrogen;
C₁₋₄ alkyl, optionally substituted with one or more fluoro;
C₃₋₆ cycloalkyl, optionally substituted with one or more fluoro;
or R³⁸ and R³⁹ or R⁴⁰ and R⁴¹ form together C₃₋₆ cycloalkyl, optionally substituted with one or more fluoro, -OH, C₁₋₄ alkyl;
or R³⁷ and R³⁸ or R³⁸ and R⁴⁰ form together C₃₋₆ cycloalkyl, optionally substituted with one or more fluoro, -OH, C₁₋₄ alkyl;
- D: is C₁₋₆ alkyl,
optionally interrupted with oxygen, sulfur or -N(R⁴²)-
and/or optionally substituted with halogen, C₃₋₆ cycloalkyl;
and/or optionally one chain carbon or two vicinal carbons form part of a C₃₋₆ cycloalkyl, wherein R⁴² is selected from the group consisting of hydrogen, C₁₋₄
alkyl, C₃₋₆ cycloalkyl and -C(O)-C₁₋₄ alkyl;
- E: is E¹, wherein E¹ is selected from the group consisting of
phenyl;
naphtyl;
heterocycle containing up to 4 heteroatoms, which are the same or different and selected from the group consisting of -O-, -S-, -S(O)-, -S(O₂)-, -N=, -N(O)= and -N(R⁴³)-; and
heterobicycle containing up to 6 heteroatoms, which are the same or different and selected from the group consisting of -O-, -S-, -S(O)-, -S(O₂)-, -N=, -N(O)= and -N(R⁴⁴)-;
wherein E¹ is optionally substituted with one or independently from each other more of
E²;
E³;
halogen;
-N(R⁴⁵R⁴⁶);
-OH;
=O, where the ring is at least partially saturated;
C₃₋₆ cycloalkyl;
-COOR⁴⁷; or
-CONR⁴⁸R⁴⁹;
and wherein R⁴³, R⁴⁴, R⁴⁵, R⁴⁶ are independently selected from the group consisting of hydrogen;
C₁₋₄ alkyl optionally substituted with -OH;
and -C(O)-C₁₋₄ alkyl optionally substituted with -OH;
and wherein R⁴⁷, R⁴⁸, R⁴⁹, are independently hydrogen or C₁₋₄ alkyl, optionally substituted with -OH;
- E²: is selected from the group consisting of E⁴, -C(O)-E⁴, -O-E⁴ and -N(R⁵⁰)-E⁴,
wherein E⁴ is phenyl or heterocycle containing up to 4 heteroatoms, which are the same or different and selected from the group consisting of -O-, -S-, -S(O)-, -S(O₂)-, -N=, -N(O)= and -N(R⁵¹)-; wherein E⁴ is optionally substituted with one or independently from each other more of
fluoro;
chloro;
cyano;
=O, where the ring is at least partially saturated;
-N(R⁵²R⁵³);
C₁₋₄ alkyl; or
-O-C₁₋₄ alkyl;
and wherein R⁵⁰, R⁵², R⁵³ are independently hydrogen or C₁₋₄ alkyl, optionally substituted with -OH;
and wherein R⁵¹ is selected from the group consisting of
hydrogen;
C₁₋₄ alkyl, optionally substituted with -OH; and
-C(O)-C₁₋₄ alkyl, optionally substituted with -OH;
- E³: is selected from the group consisting of C₁₋₆ alkyl, -O-C₁₋₆ alkyl; -N(R⁵⁴)-C₁₋₆ alkyl, wherein E³ is optionally substituted with one or independently from each other more of
fluoro;
-N(R⁵⁵R⁵⁶);
E⁵;
and/or E³ is optionally interrupted with one or more oxygen;
and wherein R⁵⁴, R⁵⁵, R⁵⁶ are independently hydrogen or C₁₋₄alkyl, optionally substituted with -OH;
- E⁵: is phenyl or heterocycle containing up to 4 heteroatoms, which are the same or different and selected from the group consisting of -O-, -S-, -S(O)-, -S(O₂)-, -N=, -N(O)= and -N(R⁵⁷)-; wherein E⁵ is optionally substituted with one or independently from each other more of
fluoro;
chloro;
cyano;
=O, where the ring is at least partially saturated;
-N(R⁵⁸R⁵⁹);
C₁₋₄ alkyl or
-O-C₁₋₄ alkyl;
and wherein R⁵⁷ is independently selected from the group consisting of hydrogen;
C₁₋₄ alkyl, optionally substituted with -OH; and
-C(O)-C₁₋₄ alkyl, optionally substituted with -OH;
and wherein R⁵⁸, R⁵⁹ are independently hydrogen or C₁₋₄ alkyl, optionally substituted with -OH.

Within the meaning of the present invention the terms are used as follows:

"Alkyl" means a straight-chain or branched carbon chain that may contain double or triple bonds.
"C₁₋₄ Alkyl" means an alkyl chain having 1 - 4 carbon atoms, e.g. at the end of a molecule methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl or amidst, e.g. -CH₂-, -CH₂-CH₂-, -CH=CH-, -CH(CH₃)-, -C(CH₂)-, -CH₂-CH₂-CH₂-, - CH(C₂H₅)-, -CH(CH₃)₂-.
"C₁₋₆ Alkyl" means an alkyl chain having 1 - 6 carbon atoms, e.g. C₁₋₄ Alkyl, methyl, ethyl, -CH=CH₂, -C≡CH, n-propyl, isopropyl, -CH=CH-CH₃, -CH₂-CH=CH₂, n-butyl, isobutyl, -CH=CH-CH₂-CH₃, -CH=CH-CH=CH₂, sec-butyl tert-butyl, n-pentane, n-hexane, or amidst, e.g. -CH₂-, -CH₂-CH₂-, -CH=CH-, -CH(CH₃)-, -C(CH₂)-, -CH₂-CH₂-CH₂-, -CH(C₂H₅)-, -CH(CH₃)₂-.
An alkyl chain "interrupted" with a heteroatom means that between two carbon atoms or at the end of the alkyl chain a heteroatom, e.g. nitrogen, oxygen or sulfur, is added. This includes for example C₁₋₄ alkyl interrupted by an oxygen atom, e.g. -CH₂-OH, - CH₂-O-CH₃, CH₂-CH₂-OH, -C₃H₆-OCH₃.
Each hydrogen of a carbon or heteroatom of the alkyl chain or interrupted alkyl chain may be replaced by a substituent.

"C₃₋₆ Cycloalkyl" means a cyclic alkyl chain having 3 - 6 carbon atoms, e.g. cyclopropyl, cyclobutyl, cyclopentyl, cyclohexyl, cyclohexenyl. Each hydrogen of a cycloalkyl carbon may be replaced by a substituent.

"Halogen" means fluoro, chloro, bromo and so called pseudo-halogens, i.e. -CN or -CNO.

"Heterocycle" means a cyclopentane, cyclohexane or cycloheptane ring that may contain up to the maximum number of double bonds (aromatic or non-aromatic ring which is fully, partially or un-saturated) wherein at least one carbon atom up to a maximum number of carbon atoms, as indicated, is replaced by a heteroatom ("containing" or "having" a heteroatom) and wherein the ring is linked to the rest of the molecule via a carbon or nitrogen atom.

"Heterobicycle" means a heterocycle which is condensed with phenyl or an additional heterocycle to form a bicyclic ring system.
"Condensed" to form a bicyclic ring means that two rings are attached to each other by sharing two ring atoms.

Preferred compounds of the formula (I) are those compounds in which one or more of the residues contained therein have the meanings given below, with all combinations of preferred substituent definitions being a subject of the present invention. With respect to all preferred compounds of the formula (I) the present invention also includes all tautomeric and stereoisomeric forms and mixtures thereof in all ratios, and their pharmaceutically acceptable salts.

In preferred embodiments of the present invention, the substituents R¹ - R³, A, B, X, G, D and E of the formula (I) independently from each other have the following meaning. Hence, one or more of the substituents R¹ - R³, A, B, X, G, D and E can have the preferred or more preferred meanings given below.

R¹ is preferably hydrogen.

R² is preferably hydrogen, chloro, -CH₃, -CH₂F, -CHF₂ or -CN.

R³ is preferably hydrogen.

Preferably in A is A¹ phenyl or heterocycle containing up to 4 heteroatoms, which are the same or different and selected from the group consisting of -O-, -S-, -S(O)-, -S(O₂)-, -N=, -N(O)= and -N(R⁴)-, wherein R⁴ has the meaning as indicated above.

More preferred, A¹ is selected from the group consisting of phenyl, pyridine, pyridine-N oxide and piperidine.

Preferably in B is Y preferably a bond or -O- and Z is preferably -C(R⁶⁰R⁶¹)-C(R⁶²R⁶³)-,
wherein
R⁶⁰, R⁶¹, R⁶², R⁶³ are independently hydrogen, fluoro, methyl, cyclopropyl or
R⁶⁰ and R⁶¹ form a cyclopropyl ring or
R⁶² and R⁶³ form a cyclopropyl ring or
R⁶⁰ and R⁶² form a cyclopropyl or cyclobutyl ring.
Preferably, R⁶⁰, R⁶¹, R⁶², R⁶³ are independently hydrogen or fluoro.

X is preferably =N-.

G is preferably -CH(R⁶⁴)-C(R⁶⁵R⁶⁶)-; wherein R⁶⁴, R⁶⁵, R⁶⁶ are independently hydrogen, methyl, -CH₂F, -CHF₂, CF₃ or cyclopropyl or R⁶⁵, R⁶⁶ form together cyclopropyl.

D is preferably -CH₂- or -CH(CH₃)- or -C(CH₃)₂- or D¹-D², where D¹ and D² are independently -CH₂-, -CH(CH₃)- or -C(CH₃)₂-.

-E is preferably selected from the group consisting of 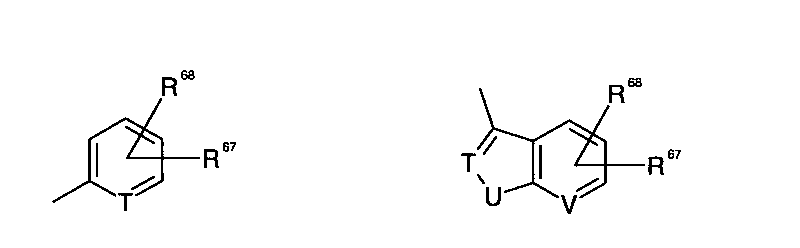 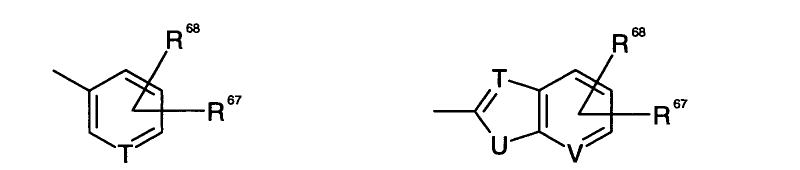 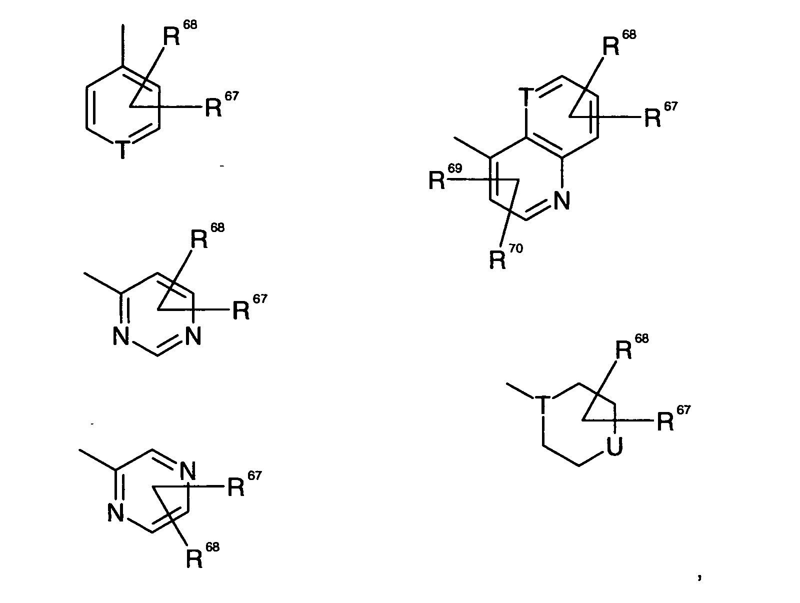 wherein
T and V are independently =CH-, =CR⁷¹- or =N-;
U is -NH-, -NR⁷²-, -O-, or -S-, wherein
R⁶⁷, R⁶⁸, R⁶⁹, R⁷⁰, R⁷¹ are independently selected from the group consisting of
hydrogen;
C₃₋₆ cycloalkyl;
E⁶;
E⁷;
halogen;
-N(R⁷³R⁷⁴);
-OH; and
-COOR⁷⁵ or -C(O)NR⁷⁶R⁷⁷;
and wherein R⁷², R⁷³, R⁷⁴, R⁷⁵, R⁷⁶, R⁷⁷ are independently
hydrogen;
C₁₋₄ alkyl; or
-C(O)-C₁₋₄ alkyl;
E⁶ is selected from the group consisting of C₁₋₆ alkyl; -O-C₁₋₆ alkyl; and -N(R⁷⁸)-C₁₋₆alkyl, wherein the C₁₋₆ alkyl group is optionally substituted with one or more of
halogen;
-N(R⁷⁹R⁸⁰);
phenyl, optionally substituted with chloro;
heterocycle containing up to 4 heteroatoms, which are the same or different and selected from the group consisting of -O-, -S-, -S(O)-, -S(O₂)-, -N=, -N(O)= and -N(R⁸¹)-, optionally substituted with chloro;
and/or E⁶ is optionally interrupted by one or more of oxygen;
and wherein R⁷⁸, R⁷⁹, R⁸⁰, R⁸¹ are independently hydrogen, C₁₋₄alkyl;
E⁷ is selected from the group consisting of E⁸; -O-E⁸; -N(R⁸²)-E⁸; and -C(O)-E⁸, wherein E⁸ is phenyl or heterocycle containing up to 4 heteroatoms, which are the same or different and selected from the group consisting of -O-, -S-, -S(O)-, -S(O₂)-, -N=, -N(O)= and -N(R⁸³)-; and wherein E⁸ is optionally substituted with chloro or -N(R⁸⁴R⁸⁵);
and wherein R⁸², R⁸³, R⁸⁴, R⁸⁵ are independently hydrogen or C₁₋₄ alkyl.

Preferably, R⁶⁷, R⁶⁸, R⁶⁹, R⁷⁰, R⁷¹ are independently hydrogen, fluoro, chloro, cyano, phenyl, chlorophenyl, methyl, amino, monomethyl amino, dimethyl amino, pyrrolyl, diazolyl, triazolyl or tertrazolyl.

Compounds of the formula (I) in which some or all of the above-mentioned groups have the preferred or more preferred meanings are also an object of the present invention.

Preferred embodiments of the compounds according to present invention are shown below:

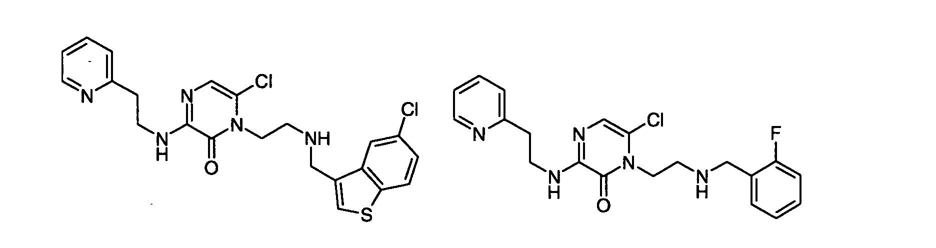

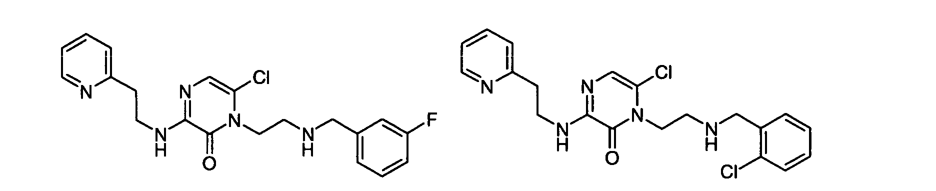

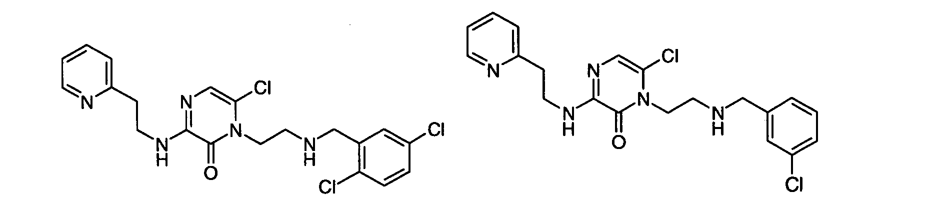

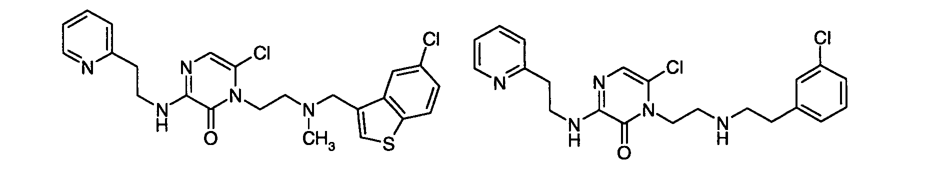

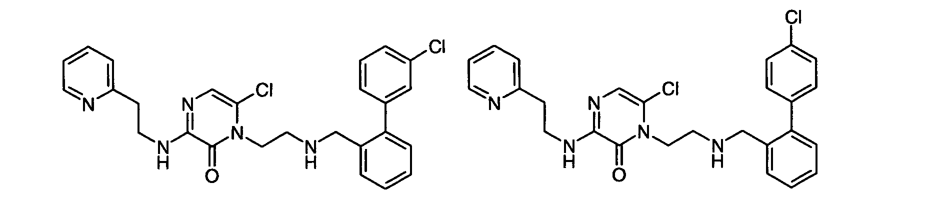

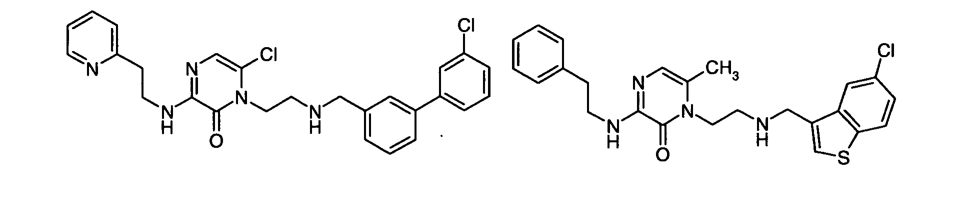

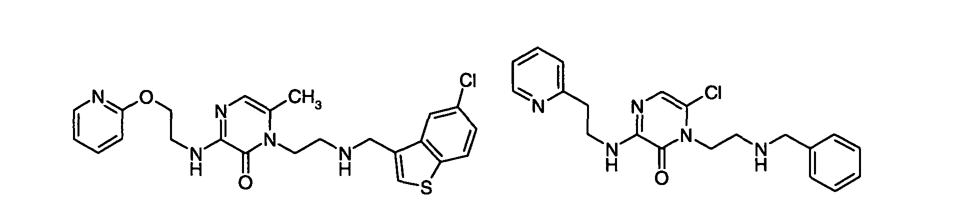

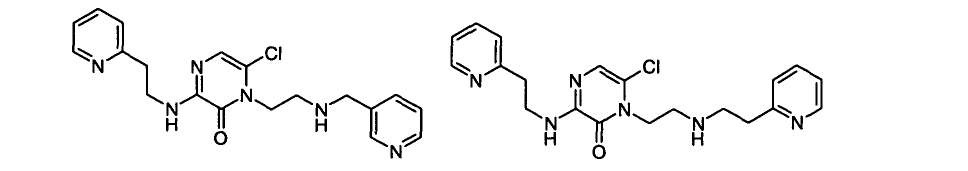

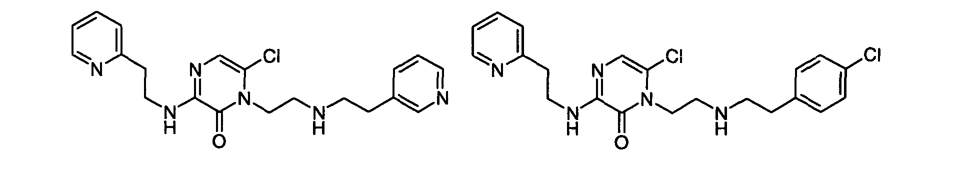

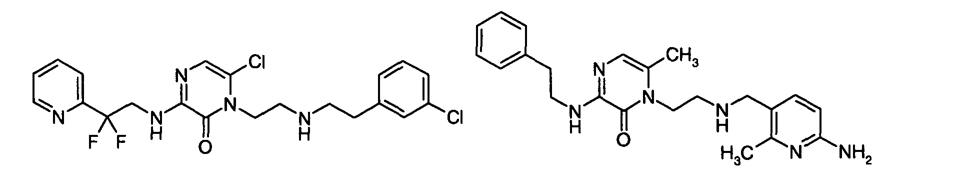

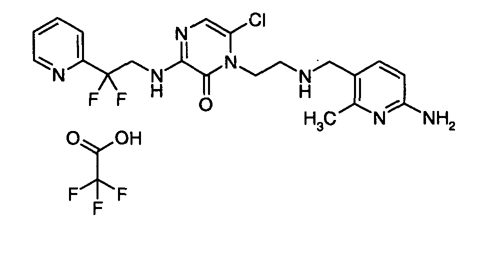

Furthermore, the present invention provides prodrugs of the compounds of the invention as described above.
"Prodrug" means a derivative that is converted into a compound according to the present invention by a reaction with an enzyme, gastric acid or the like under a physiological condition in the living body, e.g. by oxidation, reduction, hydrolysis or the like, each of which is carried out enzymatically. Examples of the prodrug are compounds, wherein the amino group in a compound of the present invention is acylated, alkylated or phosphorylated to form, e.g., eicosanoylamino, alanylamino, pivaloyloxymethylamino or wherein the hydroxyl group is acylated, alkylated, phosphorylated or converted into the borate, e.g. acetyloxy, palmitoyloxy, pivaloyloxy, succinyloxy, fumaryloxy, alanyloxy or wherein the carboxyl group is esterified or amidated. These compounds can be produced from compounds of the present invention according to well-known methods.

Where tautomerism, like e.g. keto-enol tautomerism, of compounds of general formula (I) or their prodrugs may occur, the individual forms, like e.g. the keto and enol form, are claimed separately and together as mixtures. Same applies for stereoisomers, like e.g. enantiomers, cis/trans isomers, conformers and the like.

In case the compounds according to formula (I) contain one or more acidic or basic groups, the invention also comprises their corresponding pharmaceutically or toxicologically acceptable salts, in particular their pharmaceutically utilizable salts. Thus, the compounds of the formula (I) which contain acidic groups can be present on these groups and can be used according to the invention, for example, as alkali metal salts, alkaline earth metal salts or as ammonium salts. More precise examples of such salts include sodium salts, potassium salts, calcium salts, magnesium salts or salts with ammonia or organic amines such as, for example, ethylamine, ethanolamine, triethanolamine or amino acids. Compounds of the formula (I) which contain one or more basic groups, i.e. groups which can be protonated, can be present and can be used according to the invention in the form of their addition salts with inorganic or organic acids. Examples for suitable acids include hydrogen chloride, hydrogen bromide, phosphoric acid, sulfuric acid, nitric acid, methanesulfonic acid, p-toluenesulfonic acid, naphthalenedisulfonic acids, oxalic acid, acetic acid, tartaric acid, lactic acid, salicylic acid, benzoic acid, formic acid, propionic acid, pivalic acid, diethylacetic acid, malonic acid, succinic acid, pimelic acid, fumaric acid, maleic acid, malic acid, sulfaminic acid, phenylpropionic acid, gluconic acid, ascorbic acid, isonicotinic acid, citric acid, adipic acid, and other acids known to the person skilled in the art. If the compounds of the formula (I) simultaneously contain acidic and basic groups in the molecule, the invention also includes, in addition to the salt forms mentioned, inner salts or betaines (zwitterions). The respective salts according to the formula (I) can be obtained by customary methods which are known to the person skilled in the art like, for example by contacting these with an organic or inorganic acid or base in a solvent or dispersant, or by anion exchange or cation exchange with other salts. The present invention also includes all salts of the compounds of the formula (I) which, owing to low physiological compatibility, are not directly suitable for use in pharmaceuticals but which can be used, for example, as intermediates for chemical reactions or for the preparation of pharmaceutically acceptable salts.

The present invention provides compounds of general formula (I) or their prodrugs as anticoagulants or thrombin inhibitors. This includes compounds for inhibiting thrombus formation, and inhibiting embolus formation in a mammal, inhibiting loss of blood platelets, inhibiting formation of blood platelet aggregates, inhibiting formation of fibrin. These compounds may optionally include anticoagulants, antiplatelet agents, and thrombolytic agents. The compounds can be added to blood, blood products, or mammalian organs in order to effect the desired inhibitions.

Furthermore, the invention includes compounds of formula (I) or their prodrugs or pharmaceutically acceptable salts for use as a medicament and their use for the manufacture of a medicament for the treatment or prophylaxis of thromboembolism, thrombosis, artherosclerosis, unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels.

The present invention also includes pharmaceutical compositions comprising a compound of formula (I) or their prodrugs or a mixture of compounds or prodrugs or a pharmaceutically acceptable salt thereof together with a pharmaceutically acceptable carrier. Optionally, these pharmaceutical compositions may additionally comprise one or more known anticoagulants.

The therapeutic use and method of using anticoagulants or thrombin inhibitors like the compounds of formula (I) of the present invention or their prodrugs or their use for the manufacture of a medicament are well known in the art and are described in more detail in US 2003/01582218 A1 which is herewith incorporated by reference.

Accordingly, therapies based on anticoagulants are indicated for the prevention and treatment of a variety of thrombotic conditions, particularly coronary artery and cerebrovascular disease. Those experienced in this field are readily aware of the circumstances requiring anticoagulant therapy. The term "patient" used herein is taken to mean mammals such as primates, including humans, sheep, horses, cattle, pigs, rabbits, dogs, cats, rats, and mice.

Compounds of the present invention are useful for treating or preventing venous thromboembolism (e. g. obstruction or occlusion of a vein by a detached thrombus; obstruction or occlusion of a lung artery by a detached thrombus), cardiogenic thromboembolism (e. g. obstruction or occlusion of the heart by a detached thrombus), arterial thrombosis (e. g. formation of a thrombus within an artery that may cause infarction of tissue supplied by the artery), atherosclerosis (e. g. arteriosclerosis characterized by irregularly distributed lipid deposits) in mammals, and for lowering the propensity of devices that come into contact with blood to clot blood.

Examples of venous thromboembolism which may be treated or prevented with compounds of the invention include obstruction of a vein, obstruction of a lung artery (pulmonary embolism), deep vein thrombosis, thrombosis associated with cancer and cancer chemotherapy, thrombosis inherited with thrombophilic diseases such as Protein C deficiency, Protein S deficiency, antithrombin III deficiency, and Factor V Leiden, and thrombosis resulting from acquired thrombophilic disorders such as systemic lupus erythematosus (inflammatory connective tissue disease). Also with regard to venous thromboembolism, compounds of the invention are useful for maintaining patency of indwelling catheters.

Examples of cardiogenic thromboembolism which may be treated or prevented with compounds of the invention include thromboembolic stroke (detached thrombus causing neurological affliction related to impaired cerebral blood supply), cardiogenic thromboembolism associated with atrial fibrillation (rapid, irregular twitching of upper heart chamber muscular fibrils), cardiogenic thromboembolism associated with prosthetic heart valves such as mechanical heart valves, and cardiogenic thromboembolism associated with heart disease.

Examples of arterial thrombosis include unstable angina (severe constrictive pain in chest of coronary origin), myocardial infarction (heart muscle cell death resulting from insufficient blood supply), ischemic heart disease (local anemia due to obstruction (such as by arterial narrowing) of blood supply), reocclusion during or after percutaneous transluminal coronary angioplasty, restenosis after percutaneous transluminal coronary angioplasty, occlusion of coronary artery bypass grafts, and occlusive cerebrovascular disease. Also with regard to arterial thrombosis, compounds of the present invention are useful for maintaining patency in arteriovenous cannulas.

Examples of atherosclerosis include arteriosclerosis.

Thrombin inhibition is useful not only in the anticoagulant therapy of individuals having thrombotic conditions, but is useful whenever inhibition of blood coagulation is required such as to prevent coagulation of stored whole blood and to prevent coagulation in other biological samples for testing or storage. Thus, the thrombin inhibitors can be added to or contacted with any medium containing or suspected of containing thrombin and in which it is desired that blood coagulation be inhibited, e. g., when contacting the mammal's blood with material selected from the group consisting of vascular grafts, stents, orthopedic prosthesis, cardiac prosthesis, and extracorporeal circulation systems.

Examples of devices that come into contact with blood include vascular grafts, stents, orthopedic prosthesis, cardiac prosthesis, and extracorporeal circulation systems The thrombin inhibitors of the invention can be administered in such oral forms as tablets, capsules (each of which includes sustained release or timed release formulations), pills, powders, granules, elixers, tinctures, suspensions, syrups, and emulsions. Likewise, they may be administered in intravenous (bolus or infusion), intraperitoneal, subcutaneous, or intramuscular form, all using forms well known to those of ordinary skill in the pharmaceutical arts. An effective but nontoxic amount of the compound desired can be employed as an anti-aggregation agent.

For treating ocular build up of fibrin, the compounds may be administered intraocularly or topically as well as orally or parenterally.

The compounds of the present invention can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber or other polymers manufactured by the Dow-Corning Corporation.

The compounds of the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

The compounds of the present invention may also be delivered by the use of monoclonal antibodies as individual carriers to which the compound molecules are coupled. The compounds of the present invention may also be coupled with soluble polymers as targetable drug carriers. Such polymers can include polyvinlypyrrolidone, pyran copolymer, polyhydroxy-propyl-methacrylamide-phenol, polyhydroxyethylaspartamide-phenol, or polyethyleneoxide-polylysine substituted with palmitoyl residues. Furthermore, the thrombin inhibitors may be coupled to a class of biodegradable polymers useful in achieving controlled release of a drug, for example, polylactic acid, polyglycolic acid, copolymers of polylactic and polyglycolic acid, polyepsilon caprolactone, polyhydroxy butyric acid, polyorthoesters, polyacetals, polydihydropyrans, polycyanoacrylates and cross linked or amphipathic block copolymers of hydrogels.

The dosage regimen utilizing the thrombin inhibitors is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.
Oral dosages of the compounds of the present invention, when used for the indicated effects, will range between about 0.01 mg per kg of body weight per day (mg/kg/day) to about 30 mg/kg/day, preferably 0.025-7.5 mg/kg/day, more preferably 0.1-2.5 mg/kg/day, and most preferably 0.1-0.5 mg/kg/day (unless specificed otherwise, amounts of active ingredients are on free base basis). For example, an 80 kg patient would receive between about 0.8 mg/day and 2.4 g/day, preferably 2-600 mg/day, more preferably 8-200 mg/day, and most preferably 8-40 mg/kg/day. A suitably prepared medicament for once a day administration would thus contain between 0.8 mg and 2.4 g, preferably between 2 mg and 600 mg, more preferably between 8 mg and 200 mg, and most preferably 8 mg and 40 mg, e. g., 8 mg, 10 mg, 20 mg and 40 mg. Advantageously, the compounds of the present invention may be administered in divided doses of two, three, or four times daily. For administration twice a day, a suitably prepared medicament would contain between 0.4 mg and 4 g, preferably between 1 mg and 300 mg, more preferably between 4 mg and 100 mg, and most preferably 4 mg and 20 mg, e. g., 4 mg, 5 mg, 10 mg and 20 mg.

Intravenously, the patient would receive the active ingredient in quantities sufficient to deliver between 0.025-7.5 mg/kg/day, preferably 0.1-2.5 mg/kg/day, and more preferably 0.1-0.5 mg/kg/day. Such quantities may be administered in a number of suitable ways, e. g. large volumes of low concentrations of active ingredient during one extended period of time or several times a day, low volumes of high concentrations of active ingredient during a short period of time, e. g. once a day. Typically, a conventional intravenous formulation may be prepared which contains a concentration of active ingredient of between about 0.01-1.0 mg/ml, e. g. 0.1 mg/ml, 0.3 mg/ml, and 0.6 mg/ml, and administered in amounts per day of between 0.01 ml/kg patient weight and 10.0 ml/kg patient weight, e. g. 0.1 ml/kg, 0.2 ml/kg, 0.5 ml/kg. In one example, an 80 kg patient, receiving 8 ml twice a day of an intravenous formulation having a concentration of active ingredient of 0.5 mg/ml, receives 8 mg of active ingredient per day. Glucuronic acid, L-lactic acid, acetic acid, citric acid or any pharmaceutically acceptable acid/conjugate base with reasonable buffering capacity in the pH range acceptable for intravenous administration may be used as buffers. Consideration should be given to the solubility of the drug in choosing an The choice of appropriate buffer and pH of a formulation, depending on solubility of the drug to be administered, is readily made by a person having ordinary skill in the art.

The compounds of the present invention can also be administered in intranasal form via topical use of suitable intranasal vehicles, or via transdermal routes, using those forms of transdermal skin patches well known to those of ordinary skill in that art. To be administered in the form of a transdermal delivery system, the dosage administration will, or course, be continuous rather than intermittent throughout the dosage regime.

The compounds of the present invention are typically administered as active ingredients in admixture with suitable pharmaceutical diluents, excipients or carriers (collectively referred to herein as "carrier" materials) suitably selected with respect to the intended form of administration, that is, oral tablets, capsules, elixers, syrups and the like, and consistent with convention pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water and the like. Moreover, when desired or necessary, suitable binders, lubricants, disintegrating agents and coloring agents can also be incorporated into the mixture. Suitable binders include starch, gelatin, natural sugars such as glucose or beta-lactose, corn-sweeteners, natural and synthetic gums such as acacia, tragacanth or sodium alginate, carboxymethylcellulose, polyethylene glycol, waxes and the like.

Lubricants used in these dosage forms include sodium oleate, sodium stearate, magnesium stearate, sodium benzoate, sodium acetate, sodium chloride and the like.

Disintegrators include, without limitation, starch methyl cellulose, agar, bentonite, xanthan gum and the like.

Typical uncoated tablet cores suitable for administration of thrombin inhibitors are comprised of, but not limited to, the following amounts of standard ingredients:

| Excipient | General Range (%) | Preferred Range (%) | Most Preferred Range (%) |
|---|---|---|---|
| mannitol | 10-90 | 25-75 | 30-60 |
| microcrystalline | 10-90 | 25-75 | 30-60 |
| cellulose magnesium stearate | 0.1-5.0 | 0.1-2.5 | 0.5-1.5 |

Mannitol, microcrystalline cellulose and magnesium stearate may be substituted with alternative pharmaceutically acceptable excipients.

The compounds of the present invention can also be co-administered with suitable antiplatelet agents, including, but not limited to, fibrinogen receptor antagonists (e. g. to treat or prevent unstable angina or to prevent reocclusion after angioplasty and restenosis), anticoagulants such as aspirin, thrombolytic agents such as plasminogen activators or streptokinase to achieve synergistic effects in the treatment of various vascular pathologies, or lipid lowering agents including antihypercholesterolemics (e. g. HMG CoA reductase inhibitors such as lovastatin, HMG CoA synthase inhibitors, etc.) to treat or prevent atherosclerosis. For example, patients suffering from coronary artery disease, and patients subjected to angioplasty procedures, would benefit from coadministration of fibrinogen receptor antagonists and thrombin inhibitors of the present invention. Also, compounds of the present invention enhance the efficiency of tissue plasminogen activator-mediated thrombolytic reperfusion. Compounds of the present invention may be administered first following thrombus formation, and tissue plasminogen activator or other plasminogen activator is administered thereafter.

Typical doses of thrombin inhibitors of the present invention in combination with other suitable anti-platelet agents, anticoagulation agents, or thrombolytic agents may be the same as those doses of thrombin inhibitors administered without coadministration of additional anti-platelet agents, anticoagulation agents, or thrombolytic agents, or may be substantially less that those doses of thrombin inhibitors administered without coadministration of additional anti-platelet agents, anticoagulation agents, or thrombolytic agents, depending on a patient's therapeutic needs.

Compounds of formula (I) and their prodrugs as well as their intermediates and reagents can be prepared as set forth below. The various routes and examples for the synthesis of the compounds of the present invention are non-limiting. If they are neither commercially available nor subsequently described explicitly, they can be obtained by analogy of the strategies and examples described hereinafter or by conventional synthetic procedures.

Some abbreviations that may appear in this application are as follows.

| ABBREVIATIONS | |
|---|---|
| Designation | Coupling Reagent |
| Ac₂O | Acetic anhydride |
| bs | Broad singlet |
| Boc (or BOC) | *tert*-Butoxycarbonyl |
| Boc₂O | *tert*-Butyldicarbonate |
| DAST | Diethylaminosulfurtrifluoride |
| DCE | 1,2-Dichloroethane |
| DCM | Dichloromethane |
| DMAP | 4-Dimethylaminopyridine |
| DMF | N,N-Dimethylformamide |
| Et₂O | Diethylether |
| Et₃N | Triethylamine |
| EtOAc | Ethyl acetate |
| EtOH | Ethanol |
| HPLC | High pressure liquid chromatography |
| ⁱPrOH | Isopropyl alcohol |
| MCPBA | meta-Chloroperbenzoic acid |
| MsCl | Methanesulfonyl chloride |
| OGr | Organic leaving group based on oxygen |
| PG | Protecting group |
| PPh₃ | Triphenylphosphine |
| rt | Retention time |
| Tf₂O | Trifluoromethanesulfonyl anhydride |
| TFA | Trifluoroacetic acid |
| TFAA | Trifluoroacetic acid anhydride |
| THF | Tetrahydrofuran |
| TLC | Thin layer chromatography |

Readily available starting materials may be amines having the formula (II) or (III)

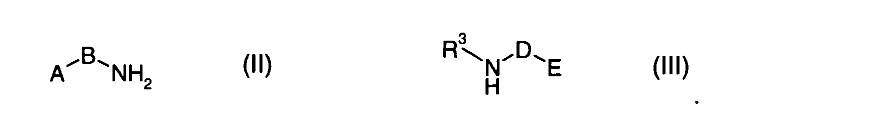

They may be purchased from commercially available sources such as Sigma-Aldrich, Fluka, ABCR or be synthesized by one skilled in the art. Common nucleophilic substitution reactions between compounds containing a suitable leaving group (e.g. halogenide, mesylate, tosylate) and nucleophiles (e.g. amines) may be employed. The conversion of diverse functional groups may allow the synthesis of various amines, e.g. conversion of esters into acids, alcohols or amides intermediates; reduction of amides, nitriles or azides to amines; also novel carbon-nitrogen palladium-catalyzed coupling reactions with suitable functionalized starting materials. For the introduction of changes in the carbon chain attached to the nitrogen atom or for the synthesis of diverse (hetero)aryl derivatives, it may be possible to make use of diverse carbon-carbon coupling reactions, e.g. transition-metal catalyzed reactions, conventional techniques for ring closure, formylation of (hetero)aryls. Schemes A through D outline general procedures for the synthesis of some compounds described below. Unless otherwise indicated in the schemes, the variables have the same meaning as described above.

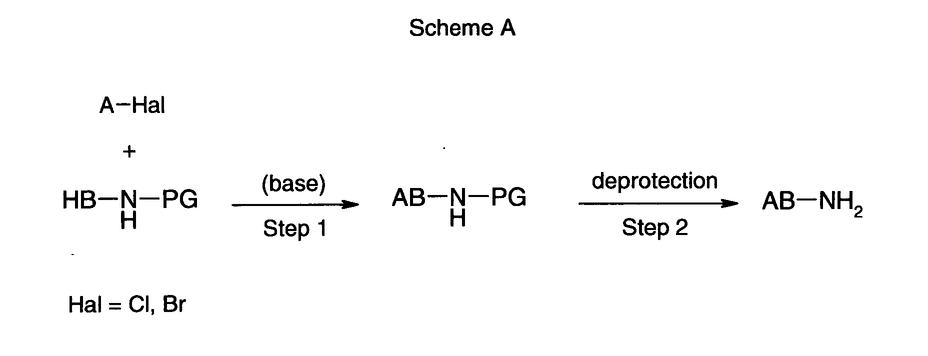

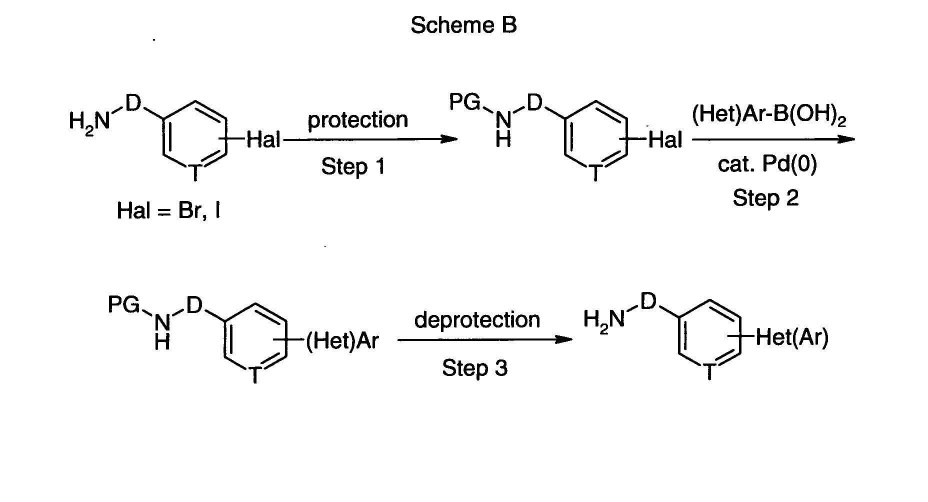

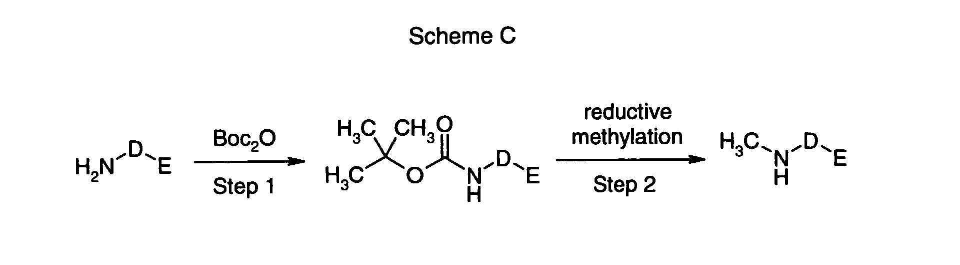

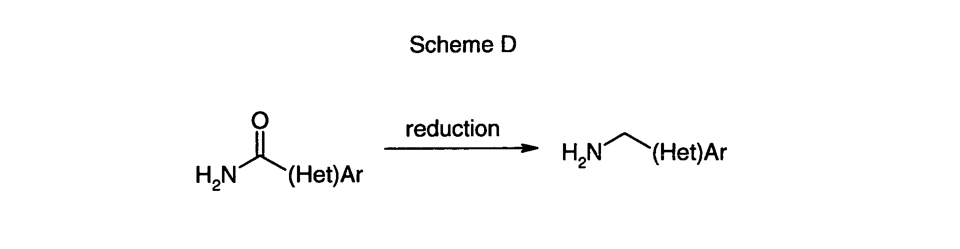

Amines having the formula (III) or (IV) 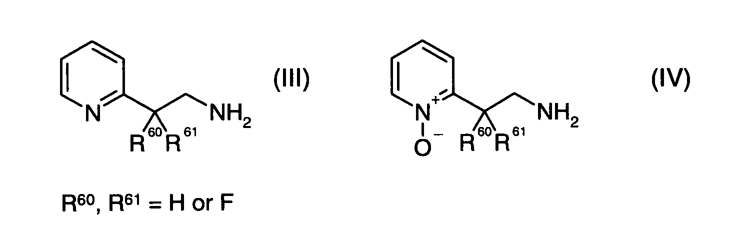 may be conveniently prepared as described in WO 01/70229 or in *Bioorg. Med. Chem. Lett*.; 13; 2003; 1353-1357 and illustrated in Scheme E. 2-Bromopyridine reacts with diethyl oxalate and *n*-butyllithium to yield ethyl 2-pyridinoylformate, which can be treated with diethylaminosulfurtrifluoride (DAST) to give a *gem-*difluorinated ethyl acetate. This can alternatively be synthesised starting from ethyl 2-pyridyl acetate through electrophilic difluorination of its potassium enolate, according to the procedure described in *J. Med. Chem.;* 46; 2003; 461-473. The ethyl difluoro-2-pyridylacetate can then be reduced to the alcohol, converted into the triflate and the azide, and finally catalytically hydrogenated to yield 2,2-difluoro-2-(2-pyridyl)ethylamine.

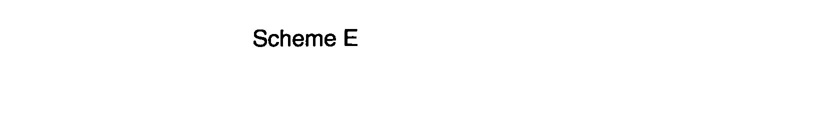

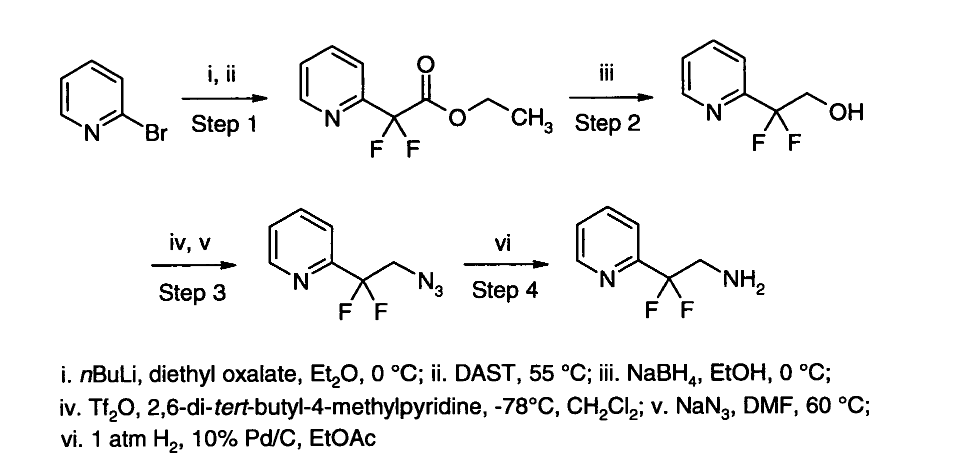

The synthesis of the 2,2-difluoro-2-(2-pyridyl-*N*-oxide)ethylamine may start with the azide, which may be prepared as outlined in Scheme E. For the oxidation of the pyridine it may be possible to follow one of the routes shown in Scheme F using *m*-chloroperbenzoic acid at elevated temperature in the presence of Kishi's radical inhibitor as in *Bioorg. Med. Chem. Lett*.; 13; 2003; 1353-1357.

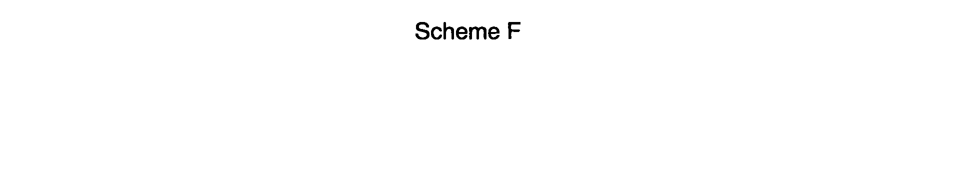

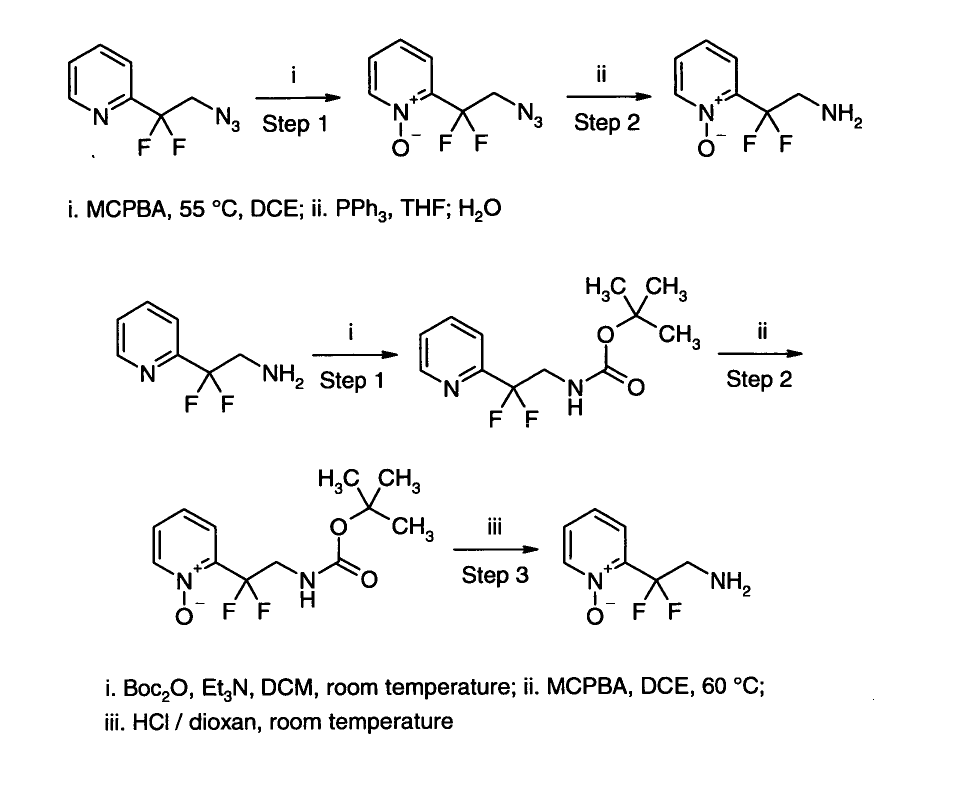

The 2*H*-pyrazin-1-yl-acetic acid ethyl esters with the formula (V) 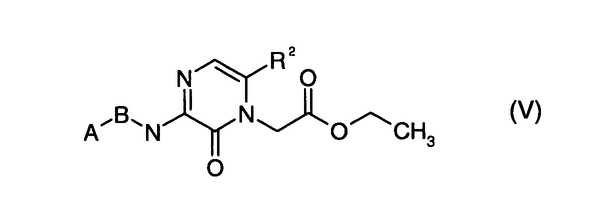 have been described in the literature. See, e.g. Schemes G through H for general procedures.
A readily scalable synthesis of the 6-methylpyrazinone is described in *Synth. Comm.;* 30; 2000; 3171-3180.

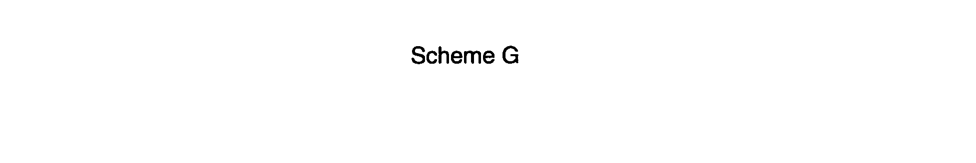

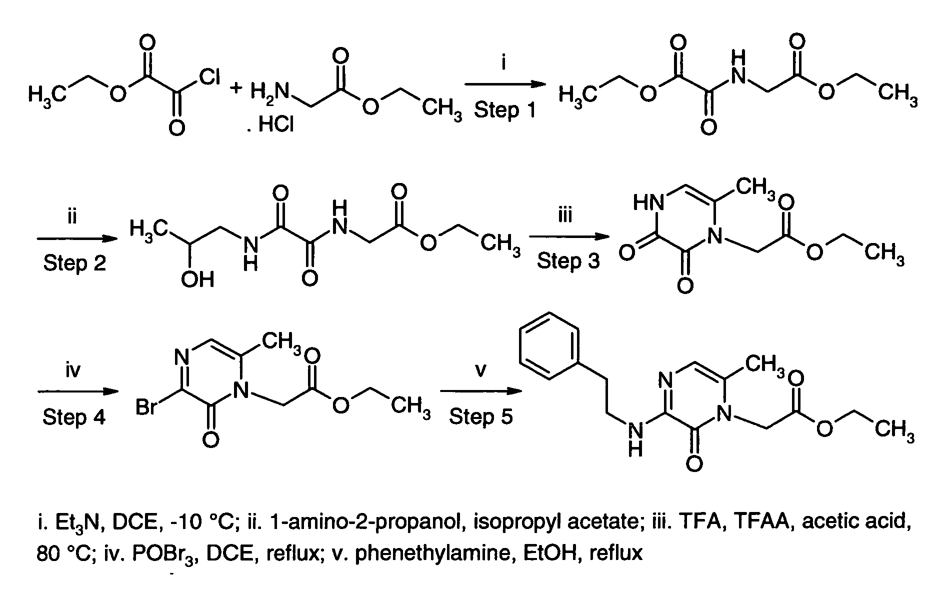

A modification of the Cheeseman pyrazinedione synthesis may be employed to obtain 1*H*-pyrazin-2-ones as described in *Bioorg. Med. Chem. Lett.;* 13; 2003; 161-164, Scheme H.

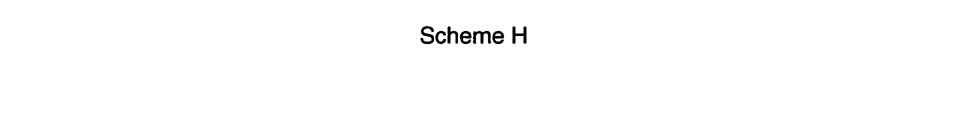

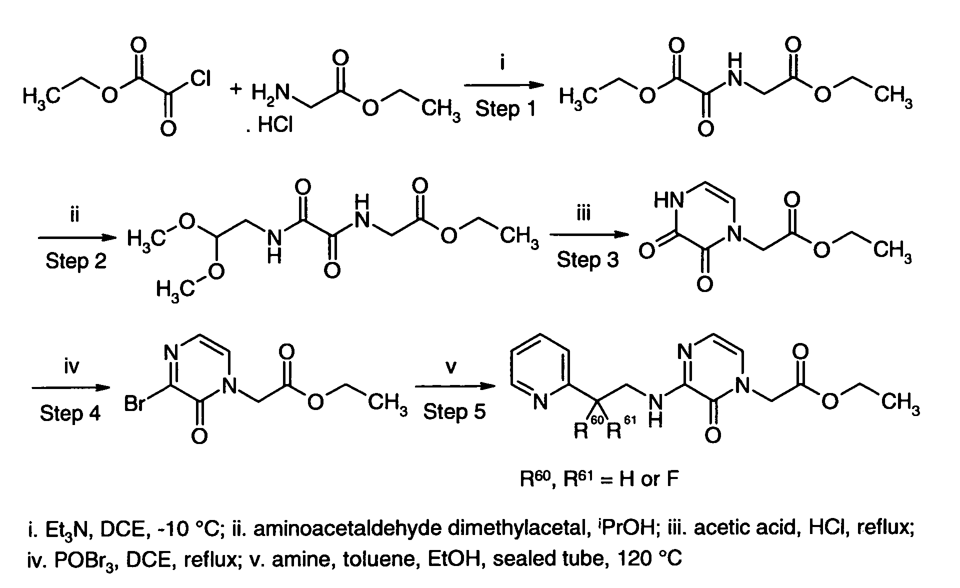

For both 6-methyl- and 1*H*-pyrazin-2-ones may be possible the synthesis of alternative intermediates with different A-B- residues using a procedure similar to the outlined above but reacting in Step 5 the intermediate bromopyrazin-2-ones with various amines.

Unless otherwise noted, all nonaqueous reactions were carried out under argon atmosphere with commercial dry solvents. Compounds were purified using flash column chromatography using Merck silica gel 60 (230-400 mesh) or reverse phase preparative HPLC using a Reprosil-Pur ODS3, 5 µm, 20 x 125 mm column with Shimadzu LCBA-Pump and SPD-10Avp UVNis diode array detector. The ¹H-NMR spectra were recorded on a Bruker AC200 (200 MHz for ¹H-NMR) or a Varian VXR-S (300 MHz for ¹H-NMR) using d₆-dimethylsulfoxide as solvent; chemical shifts are reported in ppm relative to tetramethylsilane. Analytical LC/MS was performed using Reprosil-Pur ODS3, 5 µM, 1 x 60 mm columns with a 10 min linear gradient from 5% to 95% acetonitril in water (0.1% TFA) at a flow rate of 250 µl/min; retention times are given in minutes.
LC/MS (I) runs on a LC10Advp-Pump (Shimadzu) with SPD-M10Avp UVNis diode array detector and QP2010 MS-detector in ESI+ modus with UV-detection at 214, 254 and 275 nm.

LC/MS (II) runs on a LC10Advp-Pump (Shimadzu) with SPD-10Avp dual wavelength UV-detector and QP2010 MS-detector in ESI+ modus with UV-detection at 214 and 254 nm.

### General procedure for making compounds of the invention

In general, compounds having the structure (I) 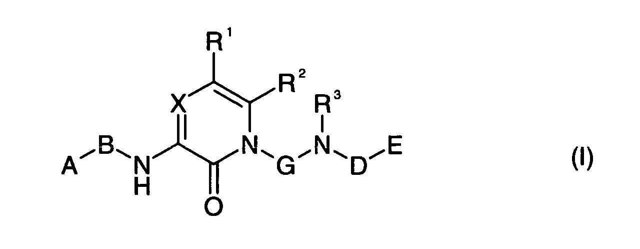 wherein the variables have the above described meanings, may be prepared by a nucleophilic substitution reaction between a substance containing a leaving group (e.g. halogenide, mesylate, tosylate) and a substance containing a nucleophilic group (e.g. amine) or by reductive amination, as shown in Scheme I.

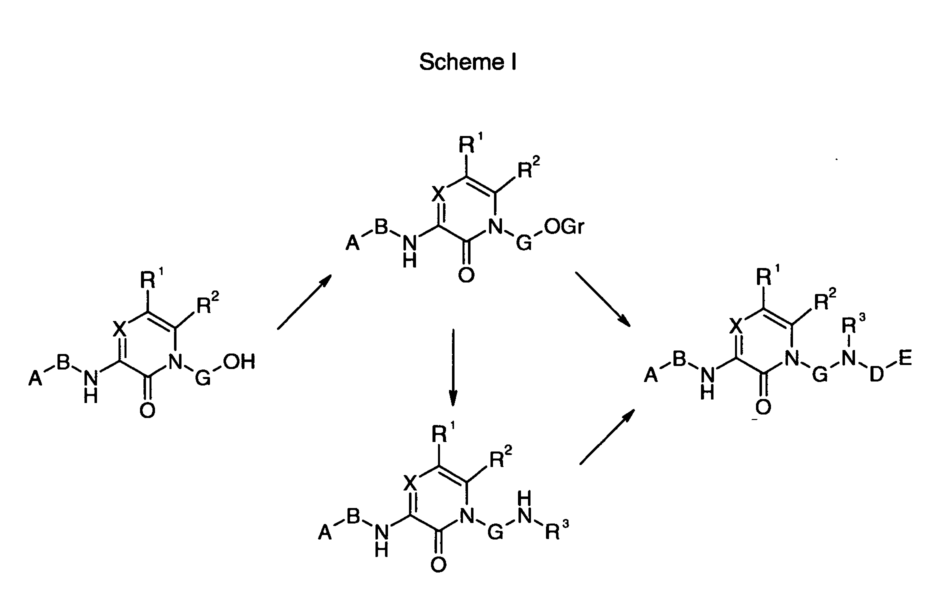

Suitable alcohol starting materials for the synthesis of the claimed compounds may be prepared according to the following procedure. As outlined in Scheme J the starting acetic acid ethyl ester is reduced in Step 1 by lithium borohydride. For the synthesis of the 6-chloro-1*H*-pyrazin-2-ones protection of the resulting alcohol is required in Step 2, e.g. by formation of an acetyl ester. Chlorination with an equimolar amount of *N*-chlorosuccinimide in Step 3 occurs with complete regioselectivity, as described in *J. Med. Chem.;* 46; 2003; 461-473. Hydrolysis of the acetate in Step 4 affords the corresponding alcohol.

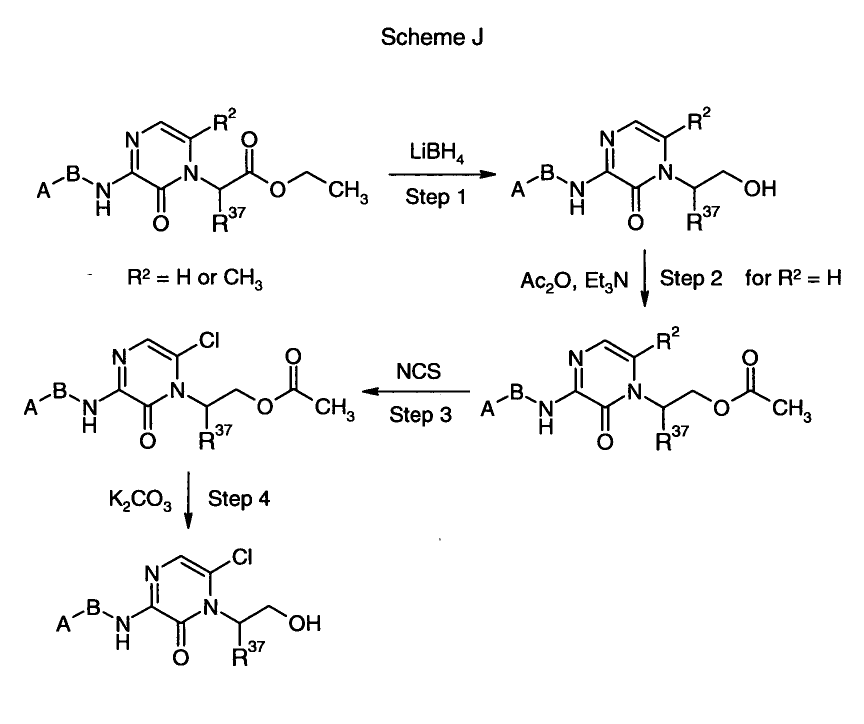

Scheme K outlines a procedure for using the alcohol formed according to Scheme J to synthesise compounds that are embodiments of the invention. In Step 1 the starting alcohol is converted into a suitable leaving group, e.g. mesylate, and nucleophilic substitution reaction in Step 2 affords the compounds object of this invention.

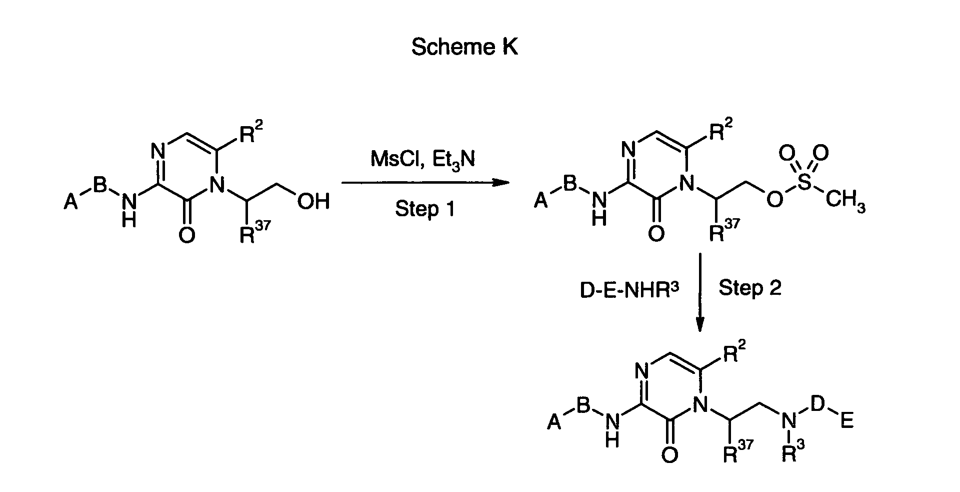

Compounds may be prepared by other means however, and the suggested starting materials and procedures described below are exemplary only and should not be considered as limiting the scope of the invention.

### PREPARATIONS

### Example 1

Procedure for making an intermediate according to Scheme A. Only Step 1 may be required in some cases to obtain the desired compounds.

### Step 1

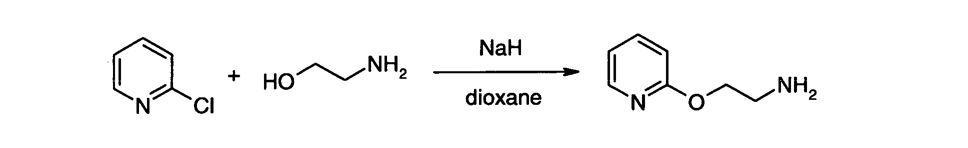

### 2-(Pyridin-2-yloxy)-ethylamine.

(For synthesis, see *Tetrahedron;* 44; 1998; 91-100) A mixture of 65 µL (2.11 mmol) and 106 mg (2.64 mmol) of sodium hydride in dioxane is refluxed for 30 min. After cooling of the solution down to room temperature, 200 mg (1.76 mmol) of 2-chloropyridine is added and the mixture is refluxed for 18 h and then concentrated under vacuum. The residue is suspended in water and extracted with dichloromethane. The organic phase is dried with sodium sulfate and concentrated to obtain the title compound as an orange oil, which was used without further purification in the next reaction step.

### Example 2

Procedure for making an intermediate according to Scheme B starting from a carbamic acid tert-butyl ester.

### Steps 2 and 3

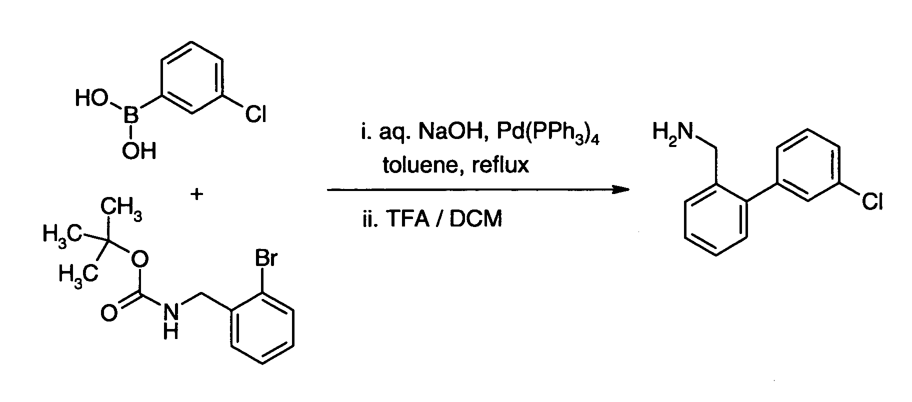

### C-(3'-Chloro-biphenyl-2-yl)-methylamine (TFA salt).

To a solution of 90.0 mg (0.580 mmol) of 3-chlorophenylboronic acid in 5 mL of toluene are added 150 µL of water, 430 µL of 5N sodium hydroxide solution, 550 µL of 2-propanol, 26.0 mg (0.022 mmol) of tetrakis(triphenylphosphine)palladium(0) and 148 mg (0.520 mmol) of 2-(bromobenzyl)-carbamic acid tert-butyl ester. The resulting mixture is refluxed under nitrogen for 2 h and then allowed to cool to room temperature. The reaction mixture is diluted with 10 mL of water, transferred to a separatory funnel, and extracted with ether. The organic phase is washed with saturated solution of sodium bicarbonate and brine, dried with sodium sulfate and the solvent is removed under reduced pressure. Purification by flash chromatography (silica gel, eluent: 2% to 5% methanol in dichloromethane) affords 109 mg of (3'-chloro-biphenyl-2-ylmethyl)-carbamic acid tert-butyl ester. The solid is dissolved in 10 mL of dichloromethane, 1.70 mL of trifluoroacetic acid is added and the solution is stirred for 1 h. After evaporation of solvents under reduced pressure, 189 mg (quant.) of the title compound in form of its trifluoroacetate salt is isolated.
¹H-NMR (300 MHz) δ = 3.95 (s, 2H), 7.26-7.59 (m, 8H), 8.13 (bs, 2H).

### Example 3

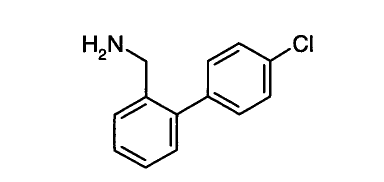

### C-(4'-Chloro-biphenyl-2-yl)-methylamine (TFA salt).

Obtained from 4-chlorophenylboronic acid and 2-(bromobenzyl)-carbamic acid tert-butyl ester using the same procedure outlined for Example 2.
¹H-NMR (300 MHz) δ=3.95 (s, 2H), 7.28-7.61 (m, 8H), 8.18 (bs, 2H).

### Example 4

Procedure for making an intermediate according to Scheme C.

### Steps 1 and 2

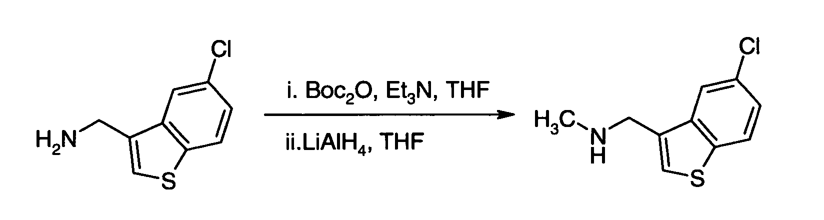

### (5-Chloro-benzo[b]thiophen-3-ylmethyl)-methyl-amine.

A solution of 100 mg (0.505 mmol) of C-(5-chloro-benzo[b]thiophen-3-yl)-methylamine, 152 mg (1.11 mmol) of di(tert-butoxycarbonyl) and 170 µL (1.21 mmol) of triethylamine in 4 mL of tetrahydrofuran is stirred at room temperature for 4 h. Solvents are removed under reduced pressure, the crude product is dissolved in 5 mL of 1N hydrochloric acid solution and extracted three times with dichloromethane. The organic phase is separated and washed with saturated sodium bicarbonate solution and brine, dried with sodium sulfate and the solvent is removed under vacuum affording 150 mg (quant.) of the (5-chloro-benzo[b]thiophen-3-ylmethyl)-carbamic acid *tert*-butyl ester.
To a solution of 20.0 mg (0.067 mmol) of the carbamic ester in 1 mL of tetrahydrofuran is added 100 µL of a 1M lithium aluminiumhydride solution in tetrahydrofuran. The reaction mixture is stirred at room temperature until gas evolution has ceased and is further heated at 65 °C for 3 h. After cooling to room temperature, 1N hydrochloric acid solution is added, followed by saturated sodium bicarbonate solution and extraction with dichloromethane. The organic phase is separated, washed with saturated sodium bicarbonate solution and brine, dried with sodium sulfate and concentrated under vacuum to obtain 14.0 mg (quant.) of the title compound.
¹H-NMR (300 MHz) δ= 2.32 (s, 3H), 3.86 (s, 2H), 7.33-7.36 (dd, 1H), 7.59 (s, 1H), 7.93-7.97 (m, 2H).
LC/MS (I) rt 2.73, m/z 212 (M+H).

### Example 5

Procedure for making an intermediate according to Scheme D.

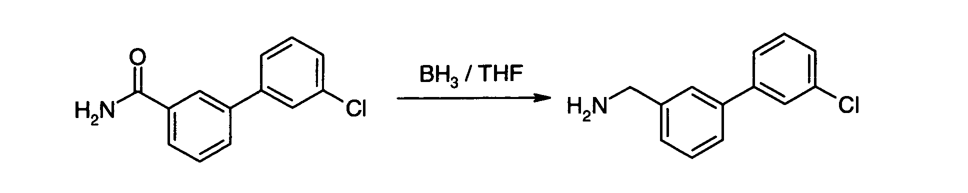

### C-(3'-Chloro-biphenyl-3-yl)-methylamine.

To a solution of 30.0 mg (0.129 mmol) of 3'-chloro-biphenyl-3-carboxylic acid amide in 4 mL of tetrahydrofuran is added 3.20 µL of a 1M borane solution in tetrahydrofuran and the resulting mixture is heated at 70 °C over night. The reaction is quenched with 2 mL of methanol and the solvents are evaporated under reduced pressure. Using preparative HPLC, 20.0 mg (71%) of the title compound is isolated.
LC/MS (I) rt 3.71, m/z 259 (M+CH₃CN+H).

### Example 6

Procedure for making an intermediate according to Scheme G, Step 5.

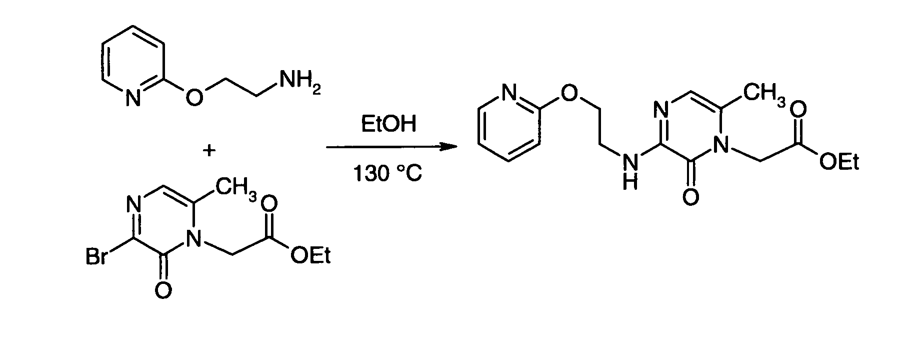

### {6-Methyl-2-oxo-3-[2-(pyridin-2-yloxy)-ethylamino]-2H-pyrazin-1yl}-acetic acid ethyl ester.

A solution of 70.0 mg (0.254 mmol) of (3-bromo-6-methyl-2-oxo-2*H*-pyrazin-1-yl)-acetic acid ethyl ester (for preparation see *Synth. Comm.;* 30; 2000; 3171-3180) and 85.2 mg (0.560 mmol) of 2-(pyridin-2-yloxy)-ethylamine in 5 mL of ethanol is heated over night at 130 °C in a sealed tube. After allowing to cool down, the solution is diluted with 10 mL of water and then extracted three times with ethyl acetate. The organic phase is separated, dried with sodium sulfate and the solvent is removed under reduced pressure. The crude mixture is purified using flash chromatography (silica gel, eluent: 50% ethyl acetate in cyclohexane) to afford 22.0 mg (26%) of the title compound.
LC/MS (I) rt 2.40, m/z 333 (M+H).

### Example 7

Procedure for making an intermediate according to Scheme J.

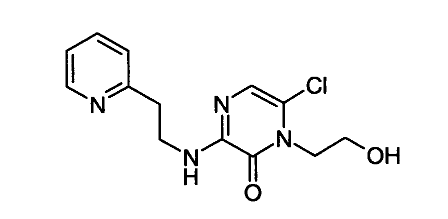

### Step 1

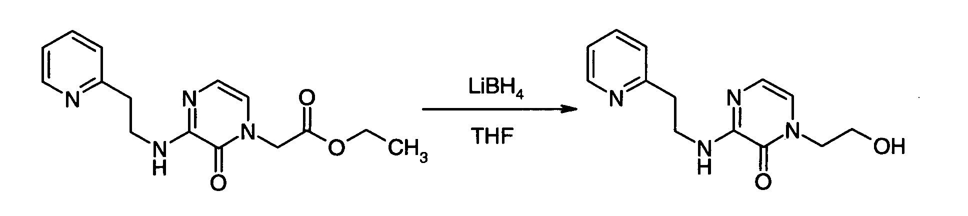

### 1-(2-Hydroxy-ethyl)-3-(2-pyridin-3-yl-ethylamino)-1H-pyrazin-2-one.

To a solution of 1.00 g (3.31 mmol) of [2-oxo-3-(2-pyridin-2-yl-ethylamino)-2*H*-pyrazin-1-yl]-acetic acid ethyl ester (see *J. Med. Chem.;* 46; 2003; 461-473 for synthesis) in 33 mL of tetrahydrofuran is added 1.65 mL (3.31 mmol) of a 2M lithium borohydride solution in tetrahydrofuran and the resulting mixture is stirred for 3 h at room temperature. After addition of 20 mL of methanol the mixture is stirred until gas evolution has ceased. The solvent is evaporated under reduced pressure and the crude product is dissolved in methanol and refluxed for 1 h. The solvent is removed under reduced pressure and the title product (861 mg, quant.) is taken directly onto the next step.
¹H-NMR (300 MHz) δ= 2.97-3.04 (m, 2H), 3.57-3.67 (m, 4H), 3.81-3.87 (m, 2H), 4.84-4.90 (m, 1H), 6.72 (s, 2H), 7.12-7.29 (m, 3H), 7.66-7.74 (m, 1H), 8.48-8.50 (m, 1H). LC/MS (I) rt 1.32, m/z 261 (M+H).

### Step 2

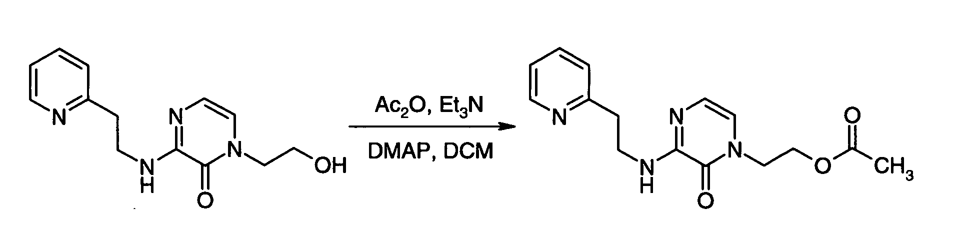

### Acetic acid 2-[2-oxo-3-(2-pyridin-2-yl-ethylamino)-2H-pyrazin-1-yl]-ethyl ester.

A solution of 596 mg (2.29 mmol) of 1-(2-hydroxy-ethyl)-3-(2-pyridin-3-yl-ethylamino)-1*H*-pyrazin-2-one, 483 µL (3.43 mmol) of triethylamine and 14.0 mg (0.115 mmol) of 4-dimethylaminopyridine is stirred for 5 min before 259 µL (2.75 mmol) of acetic anhydride is added. After stirring for 1 h at room temperature, the crude product is washed sequentially with saturated aqueous sodium bicarbonate solution, water and brine. The organic layer is dried with sodium sulfate and the solvent is removed under reduced pressure to yield 692 mg (quant.) of the title product. LC/MS (I) rt 1.97, m/z 303 (M+H).

### Step 3

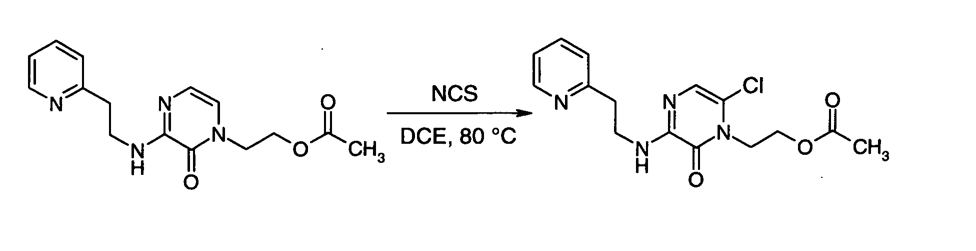

### Acetic acid 2-[6-chloro-2-oxo-3-(2-pyridin-2-yl-ethylamino)-2H-pyrazin-1-yl]-ethyl ester.

To 700 mg (2.31 mmol) of acetic acid 2-[2-oxo-3-(2-pyridin-2-yl-ethylamino)-2*H*-pyrazin-1-yl]-ethyl ester in 25 mL of 1,2-dichloroethane is added a solution of 309 mg (2.31 mmol) of *N*-chlorosuccinimide in 3 mL of 1,2-dichloroethane. The resulting mixture is heated for 90 min at 80 °C and then allowed to cool to room temperature before being washed sequentially with saturated aqueous sodium bicarbonate solution, water and brine. The organic layer is dried with sodium sulfate and the solvent is removed under reduced pressure. The crude product is purified by flash chromatography (silica gel, eluent: 20% to 100% ethyl acetate in cyclohexane) to yield 638 mg (82%) of the title compound. LC/MS (II) rt 2.36, m/z 337 (M+H).

### Step 4

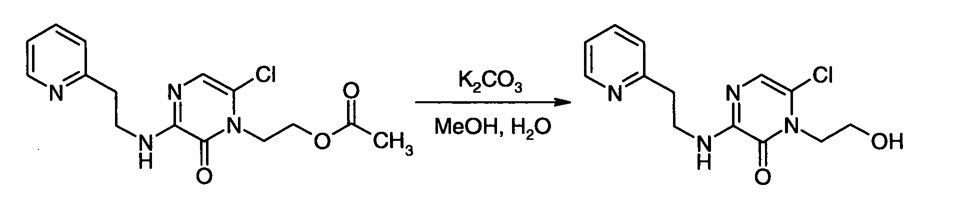

### 6-Chloro-1-(2-hydroxy-ethyl)-3-(2-pyridin-2-yl-ethylamino)-1H-pyrazin-2-one.

To 76.0 mg (0.226 mmol) of acetic acid 2-[6-chloro-2-oxo-3-(2-pyridin-2-yl-ethylamino)-2*H*-pyrazin-1-yl]-ethyl ester in 3 mL of methanol is added 1 mL of 1M potassium carbonate solution. The resulting mixture is stirred for 1 h at room temperature, then acidified with a 0.2N hydrochloric acid solution and washed once with dichloromethane. The aqueous phase is separated, neutralised with saturated aqueous sodium bicarbonate solution and extracted six times with dichloromethane. The organic phases are collected, dried with sodium sulfate and the solvent is evaporated under reduced pressure to give 66.5 mg (quant.) of the title compound.
¹H-NMR (300 MHz) δ= 2.98-3.03 (m, 2H), 3.58-3.65 (m, 4H), 4.09-4.13 (m, 2H), 4.86 (bs, 1H), 6.81 (s, 1H), 7.21-7.29 (m, 3H), 7.63-7.68 (m, 1H), 8.44-8.45 (m, 1H). LC/MS (I) rt 1.92, m/z 295 (M+H).

Using a procedure similar to the one outlined above, the following compounds were prepared.

### Example 8

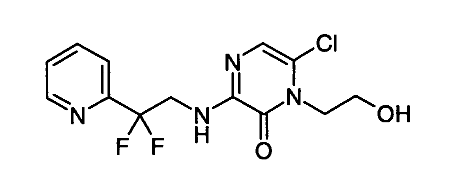

### Step 1

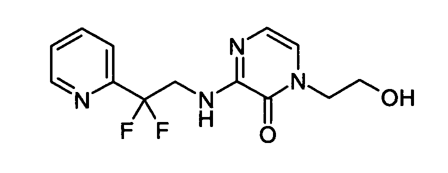

### 3-(2,2-Difluoro-2-pyridin-2-yl-ethylamino)-1-(2-hydroxy-ethyl)-1H-pyrazin-2-one.

Obtained from [3-(2,2-difluoro-2-pyridin-2-yl-ethylamino)-2-oxo-2*H*-pyrazin-1-yl]-acetic acid ethyl ester.
LC/MS (I) rt 3.25, m/z 297 (M+H).

### Step 2

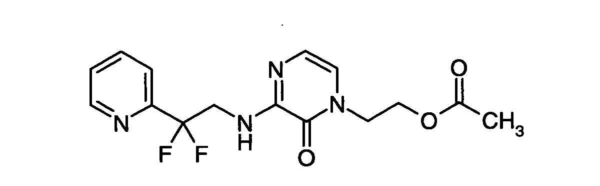

### Acetic acid 2-[3-(2,2-difluoro-2-pyridin-2-yl-ethylamino)-2-oxo-2H-pyrazin-1-yl]-ethyl ester.

LC/MS (II) rt 4.33, m/z 339 (M+H).

### Step 3

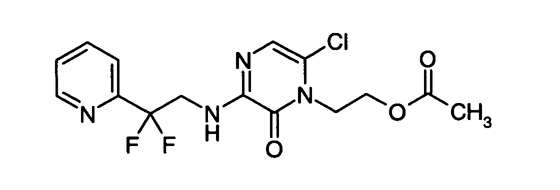

### Acetic acid 2-[6-chloro-3-(2,2-difluoro-2-pyridin-2-yl-ethylamino)-2-oxo-2H-pyrazin-1-yl]-ethyl ester (TFA salt).

¹H-NMR (300 MHz) δ= 1.95 (s, 3H), 4.15-4.30 (m, 6H), 6.88 (s, 1H), 7.26-7.30 (m, 1H), 7.50-7.54 (m, 1H), 7,63-7.66 (m, 1H), 7.91-7.96 (m, 1H), 8.63 (d, 1H).
LC/MS (II) rt 3.72, m/z 373 (M+1).

### Step 4

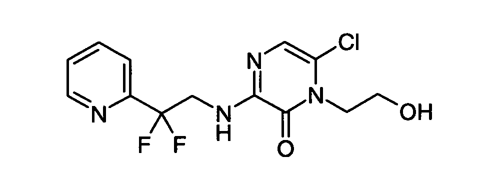

### 6-Chloro-2-(2,2-difluoro-2-pyridin-2-yl-ethylamino)-1-(2-hydroxy-ethyl)-1H-pyrazin-2-one.

¹H-NMR (300 MHz) δ= 3.57-3.63 (m, 2H), 3.78 (s, 2H), 4.10-4.27 (m, 4H), 4.86-4.90 (m, 1H), 6.68 (s, 1H), 7.18-7.22 (m, 1H), 7.50-7.54 (m, 1H), 7.64-7.67 (m, 1H), 7.91-7.96 (m, 1H), 8.66 (d, 1H).
LC/MS (I) rt 3.25, m/z 331 (M+H).

### Example 9

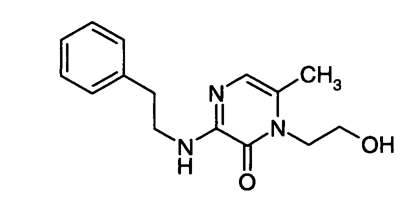

### 1-(2-Hydroxy-ethyl)-6-methyl-3-phenethylamino-1H-pyrazin-2-one.

Obtained from (6-methyl-2-oxo-3-phenethylamino-2*H*-pyrazin-1-yl)-acetic acid ethyl ester according to the procedure described for Step 1 in Scheme J (see Example 7).
¹H-NMR (300 MHz) δ= 2.19 (s, 3H), 2.81-2.86 (m, 2H), 3.44-3.51 (m, 2H), 3.57-3.62 (m, 2H), 3.91-3.95 (m, 2H), 4.82-4.84 (t, 1H), 6.59 (s, 1H), 6.65-6.68 (m, 1H), 7.13-7.27 (s, 5H).
LC/MS (II) rt 2.19, m/z 274 (M+H).

### Example 10

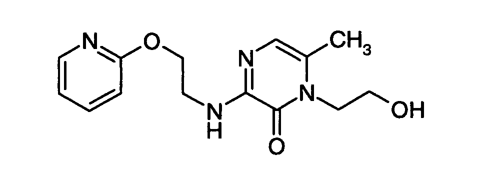

### 1-(2-Hydroxy-ethyl)-6-methyl-3-[2-(pyridin-2-yloxy)-ethylamino]-1H-pyrazin-2-one.

Obtained from {6-methyl-2-oxo-3-[2-(pyridin-2-yloxy)-ethylamino]-2H-pyrazin-1-yl}-acetic acid ethyl ester according to the procedure described for Step 1 in Scheme J (see Example 7).
The crude mixture was taken directly onto the next step.

Following examples deal with compounds of the invention synthesised according to Scheme K.
A description of the general procedure used for Step 1 follows.

### Step 1

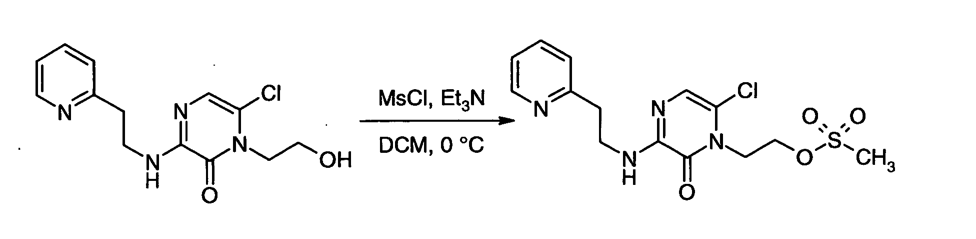

### Methanesulfonic acid 2-[6-chloro-2-oxo-3-(2-pyridin-2-yl-ethylamino)-2H-pyrazin-1yl]-ethyl ester.

After addition of 83.5 µL(0.594 mmol) of triethylamine to a solution of 25.0 mg (0.085 mmol) of 6-chloro-1-(2-hydroxy-ethyl)-3-(2-pyridin-2-yl-ethylamino)-1*H*-pyrazin-2-one in 1 mL of dichloromethane, the reaction mixture is cooled to 0 °C with an ice bath and a solution of 14.4 µL (0.187 mmol) of methanesulfonylchloride in 1 mL of dichloromethane is added. The resulting solution is stirred for 30 min and then diluted with 5 mL of dichloromethane and washed with saturated aqueous sodium bicarbonate solution and brine. The organic layer is dried with sodium sulfate and concentrated under reduced pressure. The crude product is used without further purification.
LC/MS (I) rt 2.85, m/z 373 (M+H).

For Step 2 in Scheme K, Methodes A through C may be used.

### Step 2 : Methode A

### Example 11

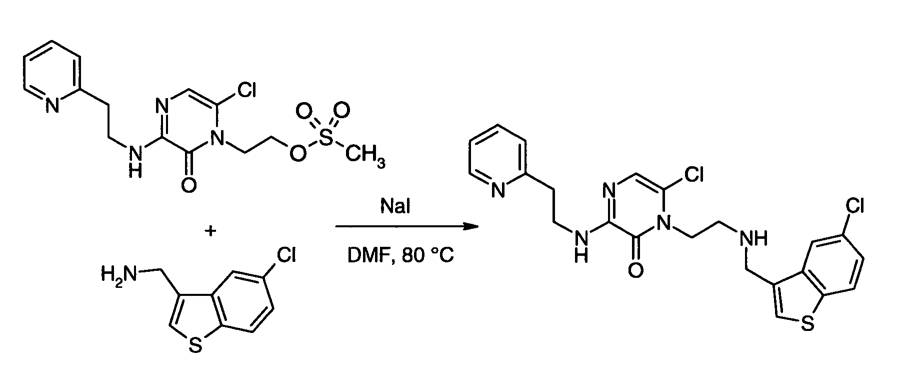

### 6-Chloro-1-{2-[(5-chloro-benzo[b]thiophen-3-ylmethyl)amino]-ethyl}-3-(2-pyridin-2-yl-ethylamino)-1H-pyrazin-2-one.

To a solution of 58.7 mg (0.157 mmol) of methanesulfonic acid 2-[6-chloro-2-oxo-3-(2-pyridin-2-yl-ethylamino)-2*H*-pyrazin-1-yl]-ethyl ester and 23.6 mg (0.157 mmol) of sodium iodide in 6 mL of *N*,*N*-dimethylformamide is added 68.5 mg (0.346 mmol) of C-(5-chloro-benzo[b]thiophen-3-yl)methylamine. The mixture is stirred 3 h at 50 °C and additionally 10 h at 80 °C. The solution is then allowed to cool to room temperature and then washed sequentially with brine and water. The aqueous phases are extracted with ethyl acetate and dichloromethane. Organic layers are collected, washed with water, dried with sodium sulfate and the solvents are removed under vacuum. The crude product is purified by flash chromatography (silica gel, eluent: 0% to 2% methanol in dichloromethane) to yield 36.6 mg (49%) of the title compound.
¹H-NMR (200 MHz) δ= 2.82-2.87 (m, 2H), 2.97-3.02 (m, 2H), 3.58-3.64 (m, 2H), 3.95 (s, 2H), 4.12-4.17 (m, 2H), 6.85 (s, 1H), 7.15-7.23 (m, 3H), 7.32-7.35 (m, 1H), 7.55 (s, 1H), 7.62-7.67 (m, 1H), 7.89-7.95 (m, 2H), 8.44-8.45 (m, 1H).
LC/MS (I) rt 2.92, m/z 474 (M+H).

### Step 2 : Methode B

### Example 12

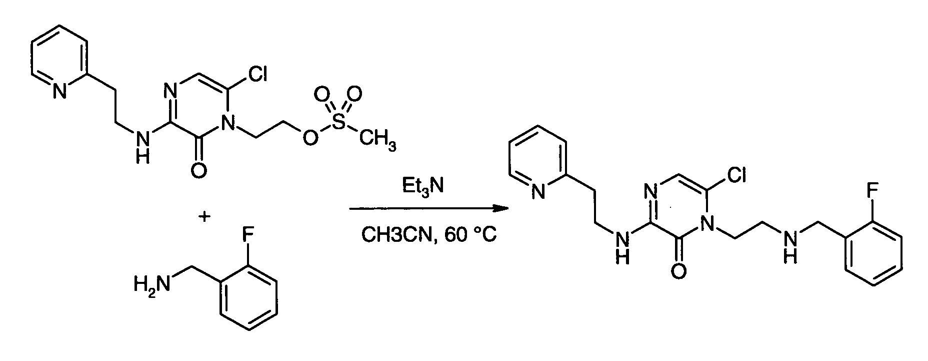

### 6-Chloro-1-[2-(2-fluoro-benzylamino)-ethyl]-3-(2-pyridin-2-yl-ethylamino)-1H-pyrazin-2-one.

To a solution of 33.6 mg (0.090 mmol) of methanesulfonic acid 2-[6-chloro-2-oxo-3-(2-pyridin-2-yl-ethylamino)-2*H*-pyrazin-1-yl]-ethyl ester in 3 mL of acetonitrile is added 25.0 µL (0.180 mmol) of triethylamin and 13.0 µL (0.110 µmol) of 2-fluorobenzylamine. The mixture is stirred overnight at 60 °C and diluted with ethyl acetate (3 mL). The organic phase is washed sequentially with water, brine and water, dried with natrium sulfate and the solvent is removed under vacuum. Purification by column chromatography (silica gel, eluent: 2% to 10% methanol in dichloromethane) affords 10.9 mg (30%) of the title compound.
¹H-NMR (300 MHz) δ=2.73-2.78 (m, 2H), 2.98-3.02 (m, 2H), 3.58-3.65 (m, 2H), 3.72 (s, 2H), 4.10-4.15 (m, 2H), 6.68 (s, 1H), 6.69-7.03 (m, 1H), 7.03-7.12 (m, 2H), 7.13-7.35 (m, 4H), 7.63-7.68 (m, 1H), 8.44 (d, 1H).
LC/MS (II) rt 2.86, m/z 402 (M+H).

Following the procedure outlined for **Example 12**, the compounds listed in the Table 1 were prepared.

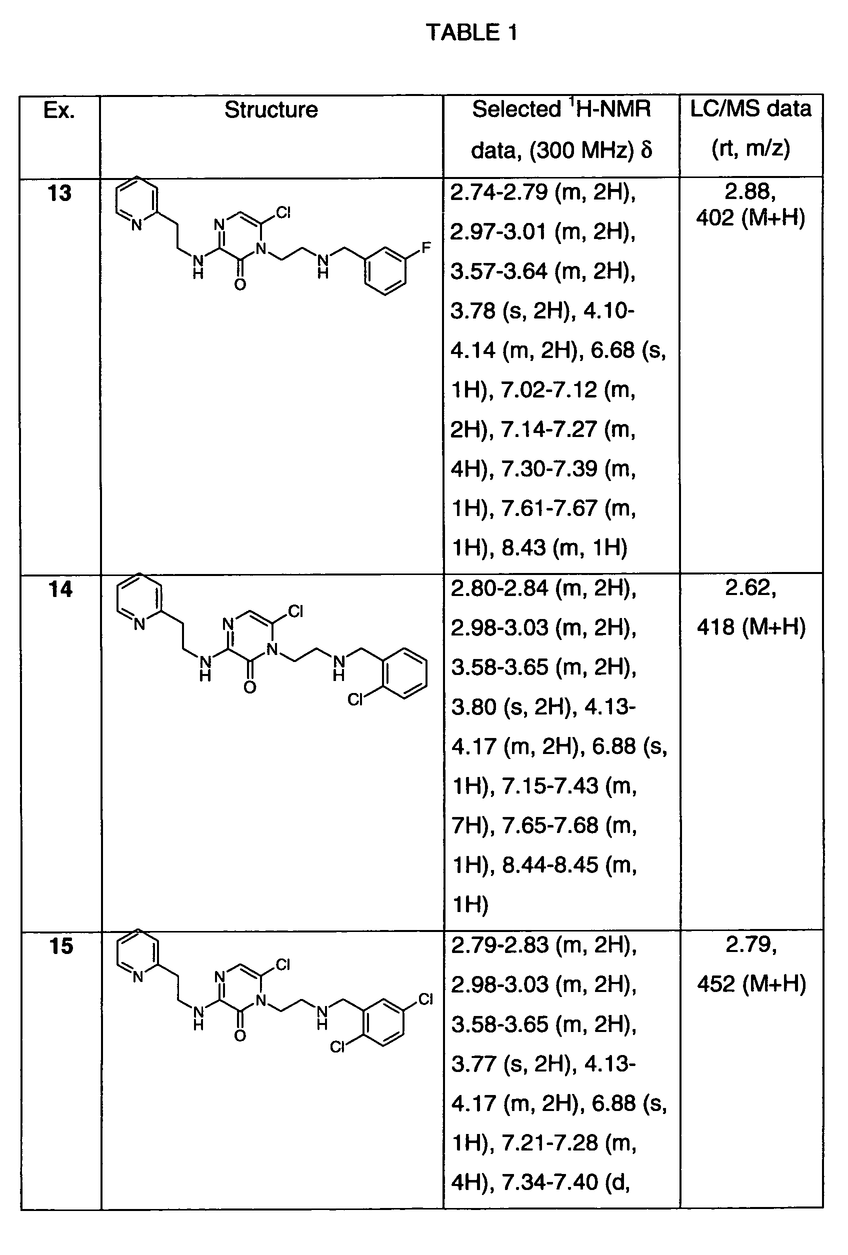

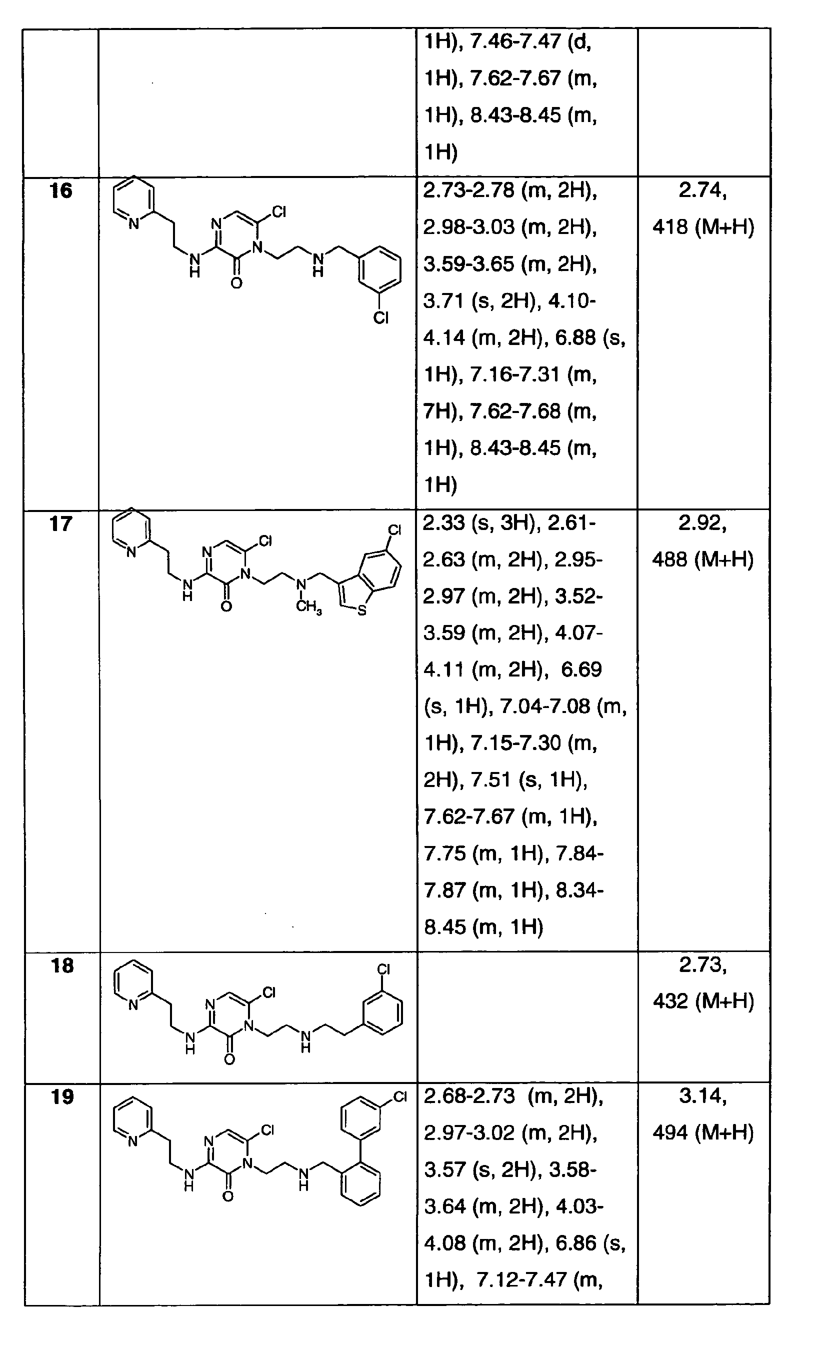

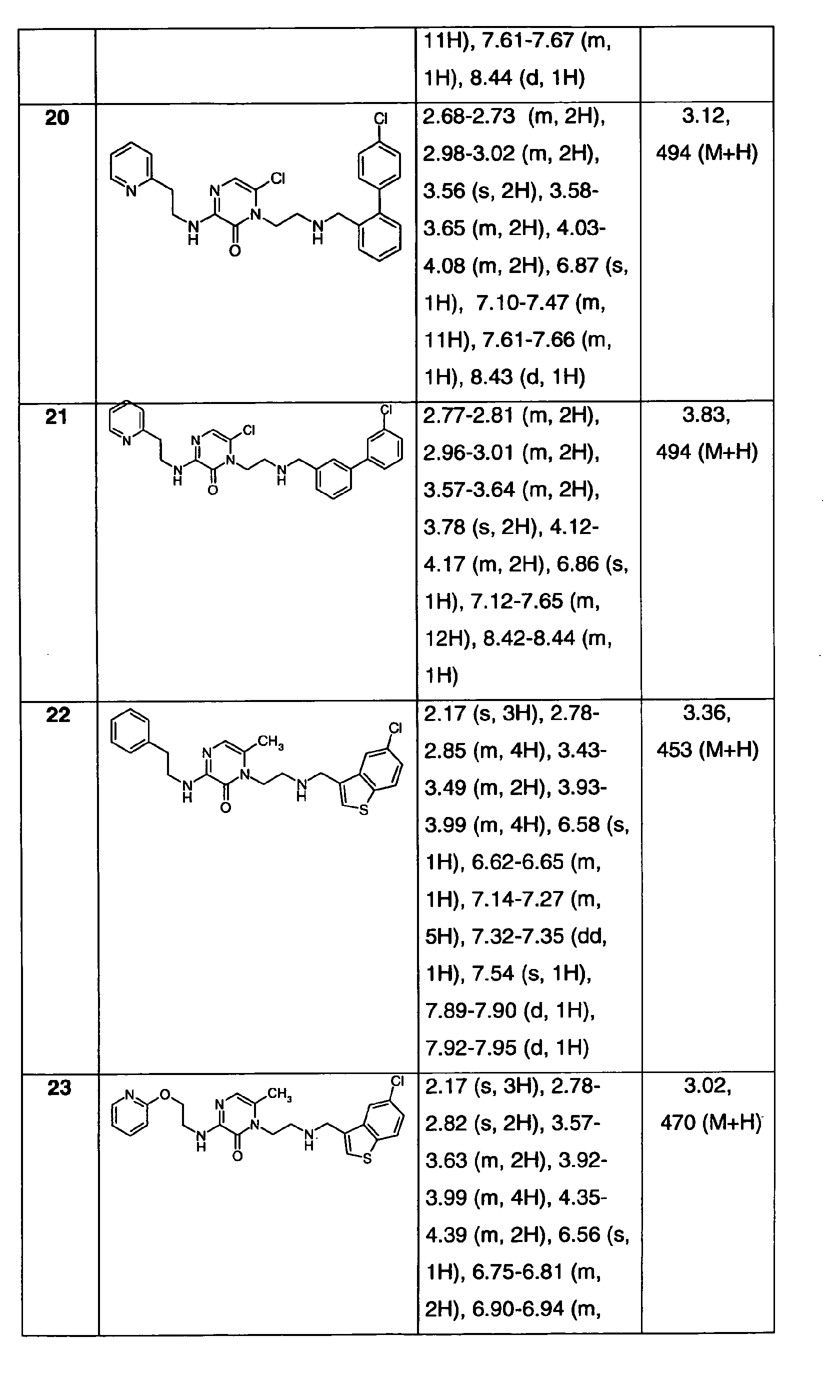

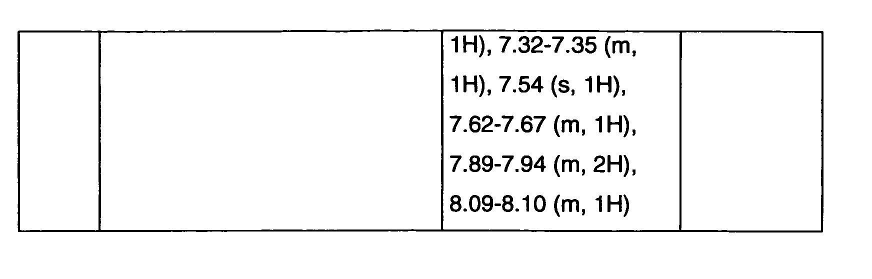

### Step 2 : Methode C

### Example 24

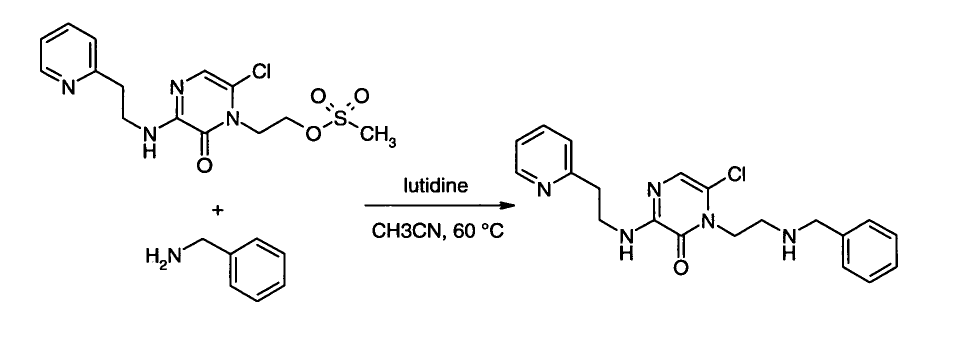

### 1-(2-Benzylamino-ethyl)-6-chloro-3-(2-pyridin-2-yl-ethylamino)-1H-pyrazin-2-one.

To a solution of 25.0 mg (0.068 mmol) of crude methanesulfonic acid 2-[6-chloro-2-oxo-3-(2-pyridin-2-yl-ethylamino)-2*H*-pyrazin-1yl]-ethyl ester in 2 mL of acetonitrile is added 11.0 µL (0.100 mmol) of benzylamine, followed by 24.0 µL (0.200 mmol) of 2,6-lutidine are added. The resulting mixture is heated to 60 °C and stirred over night. The reaction mixture is cooled down and diluted with 5 mL of dichloromethane, washed with a saturated solution of sodium hydrogencarbonate and brine, dried with sodium sulfate and evaporated under reduced pressure. The crude product is purified by flash chromatography (silica gel, eluent: 5% to 10% methanol in dichloromethane) to yield 13.1 mg (50%) of the title compound.
¹H-NMR (300 MHz) δ= 2.92-3.02 (m, 4H), 3.59-3.65 (m, 2H), 3.86 (s, 2H), 4.20-4.24 (m, 2H), 6.89 (s, 1H), 7.12-7.41 (m, 8H), 7.62-7.68 (m, 1H), 8.44 (d, 1H).
LC/MS (I) rt 2.41, m/z 384 (M+H).

Following the procedure outlined for **Example 24,** the compounds listed in the Table 2 were prepared.

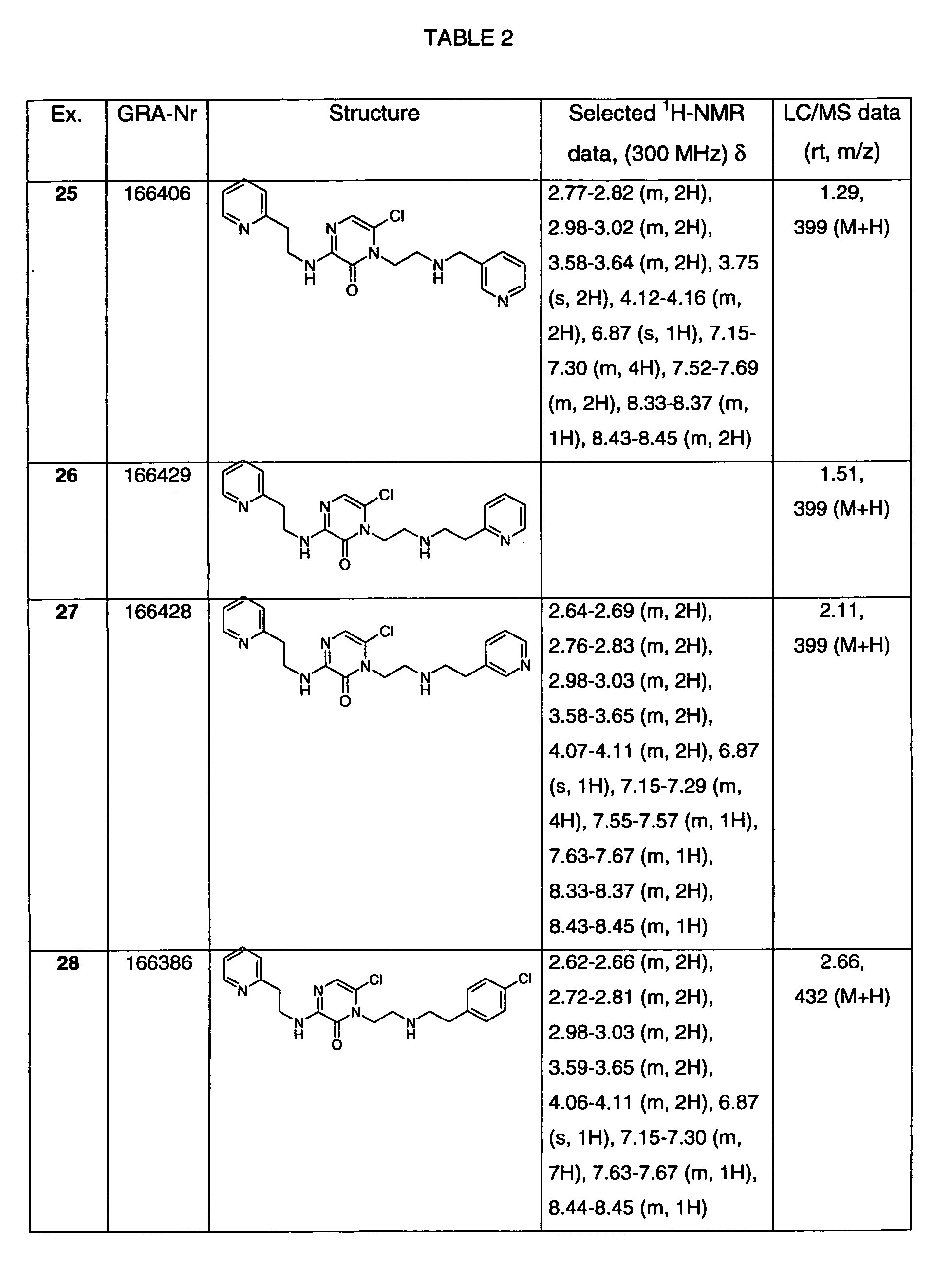

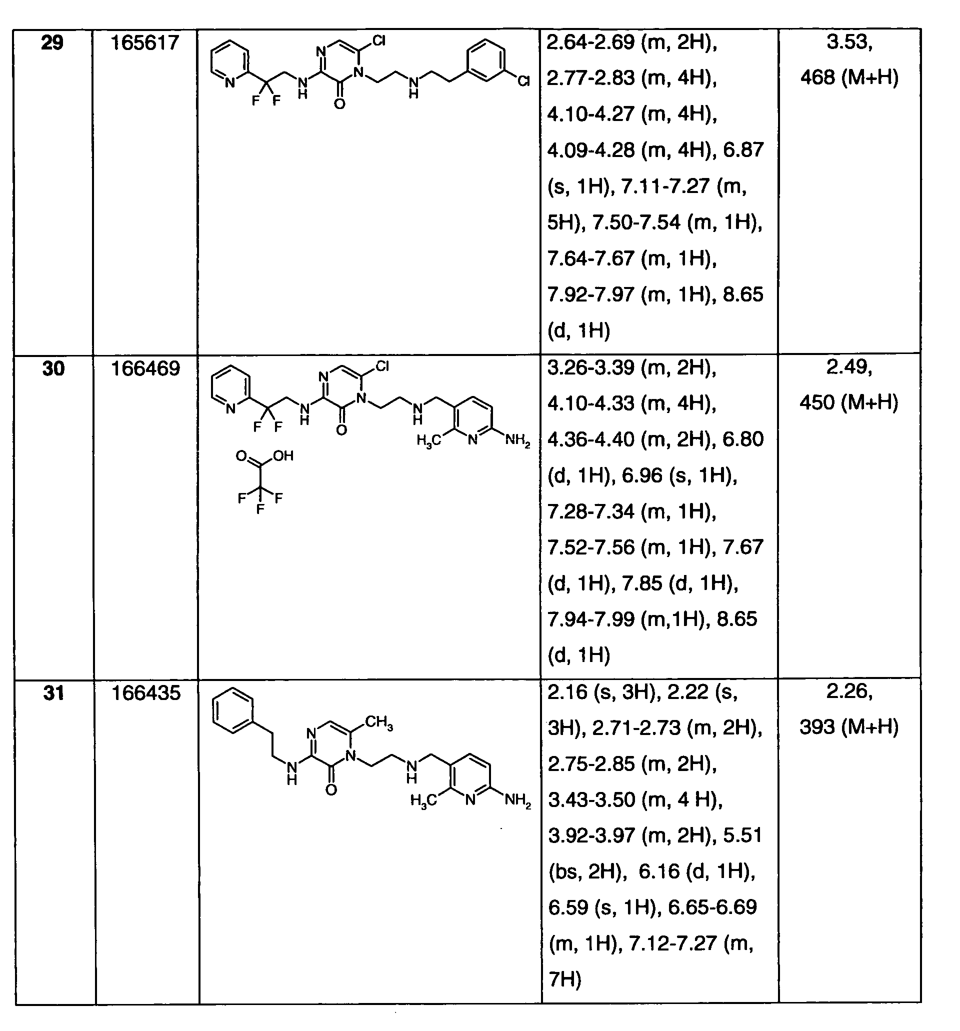

### ASSAYS

### Example 32 aPTT protocol

The aPTT measurements were carried out with an CoaData coagulometer from HelenaBioscience on 50ul human standard plasma obtained from Dade Behring. After activation with 50ul ellagic acid and cephalin using the Actin kit from Dade Behring, coagulation was triggered by addition of 50ul 25mM calcium chloride. Clotting time was measured by the instrument in seconds.

### Example 33 Kᵢ determinations thrombin

The Ki determinations were carried out at 20 °C with the fluorogenic substrate Tosyl-GPR-AMC (Bachem, Heidelberg, Germany; λ_{exc} = 370 nm, λₑₘ = 450 nm) at a thrombin concentration of 100 pM in HBS pH 7.4. The substrate was added to a final concentration of 20 µM in a total assay volume of 100 µl. The enzymatic reaction was started by addition of substrate. The emission at 450 nm was monitored in 1 minute intervals for 10 minutes using a polarstar reader (BMG Laboratories, Offenburg, Germany). Initial velocities of the control and the inhibited reactions (vₒ and vᵢ) were estimated in FU/min at different compound concentrations. The inhibition constants were calculated using the Michaelis-Menten equation for competitive inhibition.

HBS: 10 mM Hepes, 150 mM NaCl, 0.005 % Tween20, pH 7.4

The compound of example 11 shows a Kᵢ of 3 nM; the compounds of the examples 12 - 20 and 22 - 31 show a Kᵢ < 10µM.

### Example 34 Protease assays

### Factor Xa:

The Ki determinations were carried out at 20 °C with the fluorogenic substrate Boc-LGR-AMC (Bachem, Heidelberg, Germany; λ_{exc} = 370 nm, λₑₘ = 450 nm) at a fXa concentration of 1 nM in HBS pH 7.4, 5 mM CaCl₂. The substrate was added to a final concentration of 100 µM in a total assay volume of 100 µl. The enzymatic reaction was started by addition of substrate. The emission at 450 nm was monitored in 1 minute intervals for 10 minutes using a polarstar reader (BMG Laboratories, Offenburg, Germany). Initial velocities of the control and the inhibited reactions (vₒ and vᵢ) were estimated in FU/min at different compound concentrations. The inhibition constants were calculated using the Michaelis-Menten equation for competitive inhibition.

HBS: 10 mM Hepes, 150 mM NaCl, 0.005 % Tween20, pH 7.4

### Tryptase:

The Ki determinations were carried out at 20 °C with the fluorogenic substrate Boc-FSR-AMC (Bachem, Heidelberg, Germany; λ_{exc} = 370 nm, λₑₘ = 450 nm) at a Tryptase concentration of 1 nM in HBS pH 7. The substrate was added to a final concentration of 20 µM in a total assay volume of 100 µl. The enzymatic reaction was started by addition of substrate. The emission at 450 nm was monitored in 1 minute intervals for 10 minutes using a polarstar reader (BMG Laboratories, Offenburg, Germany). Initial velocities of the control and the inhibited reactions (vₒ and vᵢ) were estimated in FU/min at different compound concentrations. The inhibition constants were calculated using the Michaelis-Menten equation for competitive inhibition.

HBS: 10 mM Hepes, 150 mM NaCl, 0.005 % Tween20, pH 7

### Trpysin:

The Ki determinations were carried out at 20 °C with the fluorogenic substrate Z-GGR-AMC (Bachem, Heidelberg, Germany; λ_{exc} = 370 nm, λₑₘ = 450 nm) at a Trypsin concentration of 0.001 U/ml in TBS pH 8. The substrate was added to a final concentration of 100 µM in a total assay volume of 100 µl. The enzymatic reaction was started by addition of substrate. The emission at 450 nm was monitored in 1 minute intervals for 10 minutes using a polarstar reader (BMG Laboratories, Offenburg, Germany). Initial velocities of the control and the inhibited reactions (vₒ and vᵢ) were estimated in FU/min at different compound concentrations. The inhibition constants were calculated using the Michaelis-Menten equation for competitive inhibition.

TBS: 20 mM Tris, 150 mM NaCl, 0.005 % Tween20, pH 8

### Chymotrpysin:

The Ki determinations were carried out at 20 °C with the fluorogenic substrate H-AAF-AMC (Bachem, Heidelberg, Germany; λ_{exc} = 370 nm, λₑₘ = 450 nm) at a Chymotrpysin concentration of 1 nM in TBS pH 8. The substrate was added to a final concentration of 100 µM in a total assay volume of 100 µl. The enzymatic reaction was started by addition of substrate. The emission at 450 nm was monitored in 1 minute intervals for 10 minutes using a polarstar reader (BMG Laboratories, Offenburg, Germany). Initial velocities of the control and the inhibited reactions (vₒ and vᵢ) were estimated in FU/min at different compound concentrations. The inhibition constants were calculated using the Michaelis-Menten equation for competitive inhibition.

TBS: 20 mM Tris, 150 mM NaCl, 0.005 % Tween20, pH 8

### Elastase

The Ki determinations were carried out at 20 °C with the fluorogenic substrate MeOSuc-AAPV-AMC (Loxo, Heidelberg, Germany; λ_{exc} = 370 nm, λₑₘ = 450 nm) at an Elastase concentration of 5 nM in Hepes buffer pH 7. The substrate was added to a final concentration of 100 µM in a total assay volume of 100 µl. The enzymatic reaction was started by addition of substrate. The emission at 450 nm was monitored in 1 minute intervals for 10 minutes using a polarstar reader (BMG Laboratories, Offenburg, Germany). Initial velocities of the control and the inhibited reactions (vₒ and vᵢ) were estimated in FU/min at different compound concentrations. The inhibition constants were calculated using the Michaelis-Menten equation for competitive inhibition.

Hepes buffer: 10 mM Hepes, 50 mM NaCl, 0.005 % Tween20, pH 7

### Plasmin

The Ki determinations were carried out at 20 °C with the fluorogenic substrate H-D-ALK-AMC (Bachem, Heidelberg, Germany; λ_{exc} = 370 nm, λₑₘ = 450 nm) at a plasmin concentration of 1 nM in HBS pH 7.4, 5 mM CaCl2. The substrate was added to a final concentration of 100 µM in a total assay volume of 100 µl. The enzymatic reaction was started by addition of substrate. The emission at 450 nm was monitored in 1 minute intervals for 10 minutes using a polarstar reader (BMG Laboratories, Offenburg, Germany). Initial velocities of the control and the inhibited reactions (vₒ and vᵢ) were estimated in FU/min at different compound concentrations. The inhibition constants were calculated using the Michaelis-Menten equation for competitive inhibition.

HBS: 10 mM Hepes, 150 mM NaCl, 0.005 % Tween20, pH 7.4

### Example 35 Selectivity profile

Table 3 lists Kᵢ values for related proteases determined in assays as described in example 34 for the compound of example 11 and demonstrate the high degree of selectivity for the inhibition of thrombin compared to the other related proteases.

**TABLE 3**

| | Thrombin | Trypsin | Factor Xa | Plasmin | Chymotrypsin | Elastase | Tryptase |
|---|---|---|---|---|---|---|---|
| Kᵢ (µM) | 0,003 | 600 | 40 | >1000 | >10 | >1000 | >1000 |

## Claims

1. A compound of formula (I) 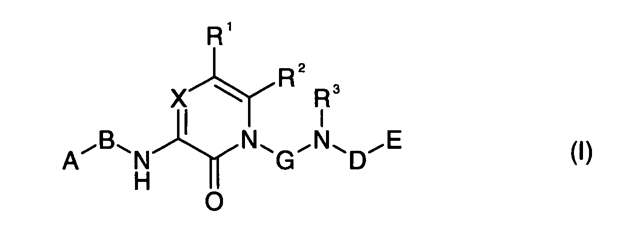 or a pharmaceutically acceptable salt thereof, wherein:
R¹ is hydrogen;
halogen; or
C₁₋₄ alkyl, optionally substituted with one or more fluoro;
R² is hydrogen;
halogen;
C₁₋₆ alkyl, optionally substituted with one or more fluoro;
C₃₋₆ cycloalkyl; or
O-C₁₋₄ alkyl;
R³ is hydrogen;
C₁₋₄ alkyl; or
C₃₋₆ cycloalkyl;
A is A¹, wherein A¹ is selected from the group consisting of:
phenyl;
naphtyl;
heterocycle containing up to 4 heteroatoms, which are the same or different and selected from the group consisting of -O-, -S-, -S(O)-, -S(O₂)-, -N=, -N(O)= and -N(R⁴)-; and
heterobicycles containing up to 6 heteroatoms, which are the same or different and selected from the group consisting of -O-, -S-, -S(O)-, -S(O₂)-, -N=, -N(O)= and -N(R⁴)-;
wherein A¹ is optionally substituted with one or independently from each other more of
A²;
A³;
halogen;
-N(R⁵R⁶);
-OH;
=O, where the ring is at least partially saturated;
C₃₋₆ cycloalkyl;
-COOR⁷; or
-CONR⁸R⁹;
and wherein R⁴, R⁵, R⁶ are independently selected from the group consisting of hydrogen, C₁₋₄ alkyl and -C(O)-C₁₋₄ alkyl;
and wherein R⁷, R⁸, R⁹, are independently hydrogen or C₁₋₄ alkyl;
A² is selected from the group consisting of A⁴, -O-A⁴ and -N(R¹⁰)-A⁴,
wherein A⁴ is phenyl or a heterocycle containing up to 4 heteroatoms, which are the same or different and selected from the group consisting of -O-, -S-, -S(O)-, -S(O₂)-, -N=, -N(O)= and -N(R¹¹)-; wherein A⁴ is optionally substituted with one or independently from each other more of
fluoro;
chloro;
-N(R¹²R¹³)
C₁₋₄ alkyl or -O-C₁₋₄ alkyl, both optionally substituted with one or
independently from each other more of fluoro or -N(R¹⁴R¹⁵);
and wherein R¹⁰, R¹², R¹³, R¹⁴, R¹⁵ are independently hydrogen or C₁₋₄ alkyl;
and wherein R¹¹ is selected from the group consisting of hydrogen, C₁₋₄ alkyl and -C(O)-C₁₋₄ alkyl;
A³ is selected from the group consisting of C₁₋₆ alkyl, -O-C₁₋₆ alkyl and -N(R¹⁶)-C₁₋₆ alkyl, wherein the C₁₋₆ alkyl group is optionally substituted with one or independently from each other more of
fluoro;
-N(R¹⁷R¹⁸);
A⁵;
and/or A³ is optionally interrupted with one or more oxygen;
and wherein R¹⁶, R¹⁷, R¹⁸ are independently hydrogen or C₁₋₄alkyl;
A⁵ is phenyl or a heterocycle containing up to 4 heteroatoms, which are the same or different and selected from the group consisting of -O-, -S-, -S(O)-, -S(O₂)-, -N=, -N(O)= and -N(R¹⁹)-; wherein A⁵ is optionally substituted with one or independently from each other more of
fluoro;
chloro;
-N(R²⁰R²¹)
C₁₋₄ alkyl or -O-C₁₋₄ alkyl, both optionally substituted with one or independently from each other more of fluoro or -N(R²²R²³);
and wherein R¹⁹ is selected from the group consisting of hydrogen, C₁₋₄ alkyl and -C(O)-C₁₋₄ alkyl;
and wherein R²⁰, R²¹, R²², R²³ are independently hydrogen or C₁₋₄ alkyl;
B is selected from the group consisting of -Y-Z-; -Y-Z-C(O)-; -Y-Z-O-C(O)-; -Y-Z-S(O)₂-; and -Y-Z-NH-C(O)- wherein
Y is a bond, -O-, -S-, -N(R²⁴)-, -N(R²⁵)-C(O)-, -C(O)-N(R²⁶)-, or -C(O)-;
Z is C₁₋₆ alkyl,
optionally interrupted with oxygen, sulfur or -N(R²⁷)-
and/or optionally substituted with one or independently from each other more of
halogen;
C₃₋₆ cycloalkyl;
-COOR²⁸;
-CON(R²⁹R³⁰)
and/or optionally one chain carbon forms part of a C₃₋₆ cycloalkyl;
and wherein R²⁴, R²⁵, R²⁶, R²⁷, R²⁸, R²⁹, R³⁰ are independently
hydrogen; or
C₁₋₄ alkyl, optionally substituted with -COOR³¹ or -CON(R³²R³³)
wherein R³¹, R³², R³³ are independently hydrogen or C₁₋₄ alkyl;
X is =C(R³⁴)- or =N-, wherein R³⁴ is
hydrogen;
C₁₋₆ alkyl, optionally substituted with one or more fluoro; or
-S(O)₂R³⁵, wherein R³⁵ is selected from the group consisting of X¹, C₁₋₆ alkyl,
and -C₁₋₆ alkyl-X¹; wherein R³⁵ is optionally substituted with one or independently from each other more of
fluoro;
chloro;
C₁₋₄ alkyl; or
-O-C₁₋₄ alkyl;
X¹ is phenyl or heterocycle containing up to 4 heteroatoms, which are the same or different and selected from the group consisting of -O-, -S-, -S(O)-, -S(O₂)-, -N=, -N(O)= and -N(R³⁶)-; and wherein R³⁶ is selected from the group consisting of hydrogen, C₁₋₄ alkyl and -C(O)-C₁₋₄ alkyl;
G is -CH(R³⁷)-C(R³⁸R³⁹)-;
-CH(R³⁷)-C(R³⁸R³⁹)-C(R⁴⁰R⁴¹)-;
wherein R³⁷, R³⁸, R³⁹, R⁴⁰, R⁴¹ are independently
hydrogen;
C₁₋₄ alkyl, optionally substituted with one or more fluoro;
C₃₋₆ cycloalkyl, optionally substituted with one or more fluoro;
or R³⁸ and R³⁹ or R⁴⁰ and R⁴¹ form together C₃₋₆ cycloalkyl, optionally substituted with one or more fluoro, -OH, C₁₋₄ alkyl;
or R³⁷ and R³⁸ or R³⁸ and R⁴⁰ form together C₃₋₆ cycloalkyl, optionally substituted with one or more fluoro, -OH, C₁₋₄ alkyl;
D is C₁₋₆ alkyl,
optionally interrupted with oxygen, sulfur or -N(R⁴²)-
and/or optionally substituted with halogen, C₃₋₆ cycloalkyl;
and/or optionally one chain carbon or two vicinal carbons form part of a C₃₋₆ cycloalkyl, wherein R⁴² is selected from the group consisting of hydrogen, C₁₋₄ alkyl, C₃₋₆ caycloalkyl and -C(O)-C₁₋₄ alkyl;
E is E¹, wherein E¹ is selected from the group consisting of
phenyl;
naphtyl;
heterocycle containing up to 4 heteroatoms, which are the same or different and selected from the group consisting of -O-, -S-, -S(O)-, -S(O₂)-, -N=, -N(O)= and -N(R⁴³)-; and
heterobicycle containing up to 6 heteroatoms, which are the same or different and selected from the group consisting of -O-, -S-, -S(O)-, -S(O₂)-, -N=, -N(O)= and -N(R⁴⁴)-;
wherein E¹ is optionally substituted with one or independently from each other more of
E²;
E³;
halogen;
-N(R⁴⁵R⁴⁶);
-OH;
=O, where the ring is at least partially saturated;
C₃₋₆ cycloalkyl;
-COOR⁴⁷; or
-CONR⁴⁸R⁴⁹;
and wherein R⁴³, R⁴⁴, R⁴⁵, R⁴⁶ are independently selected from the group consisting of hydrogen;
C₁₋₄ alkyl optionally substituted with -OH;
and -C(O)-C₁₋₄ alkyl optionally substituted with -OH;
and wherein R⁴⁷, R⁴⁸, R⁴⁹, are independently hydrogen or C₁₋₄ alkyl, optionally substituted with -OH;
E² is selected from the group consisting of E⁴, -C(O)-E⁴, -O-E⁴ and -N(R⁵⁰)-E⁴,
wherein E⁴ is phenyl or heterocycle containing up to 4 heteroatoms, which are the same or different and selected from the group consisting of -O-, -S-, -S(O)-, -S(O₂)-, -N=, -N(O)= and -N(R⁵¹)-; wherein E⁴ is optionally substituted with one or independently from each other more of
fluoro;
chloro;
cyano;
=O, where the ring is at least partially saturated;
-N(R⁵²R⁵³);
C₁₋₄ alkyl; or
-O-C₁₋₄ alkyl;
and wherein R⁵⁰, R⁵², R⁵³ are independently hydrogen or C₁₋₄ alkyl, optionally substituted with -OH;
and wherein R⁵¹ is selected from the group consisting of
hydrogen;
C₁₋₄ alkyl, optionally substituted with -OH; and
-C(O)-C₁₋₄ alkyl, optionally substituted with -OH;
E³ is selected from the group consisting of C₁₋₆ alkyl, -O-C₁₋₆ alkyl; -N(R⁵⁴)-C₁₋₆ alkyl, wherein E³ is optionally substituted with one or independently from each other more of
fluoro;
-N(R⁵⁵R⁵⁶);
E⁵;
and/or E³ is optionally interrupted with one or more oxygen;
and wherein R⁵⁴, R⁵⁵, R⁵⁶ are independently hydrogen or C₁₋₄alkyl, optionally substituted with -OH;
E⁵ is phenyl or heterocycle containing up to 4 heteroatoms, which are the same or different and selected from the group consisting of -O-, -S-, -S(O)-, -S(O₂)-, -N=, -N(O)= and -N(R⁵⁷)-; wherein E⁵ is optionally substituted with one or independently from each other more of
fluoro;
chloro;
cyano;
=O, where the ring is at least partially saturated;
-N(R⁵⁸R⁵⁹);
C₁₋₄ alkyl or
-O-C₁₋₄ alkyl;
and wherein R⁵⁷ is independently selected from the group consisting of hydrogen;
C₁₋₄ alkyl, optionally substituted with -OH; and
-C(O)-C₁₋₄ alkyl, optionally substituted with -OH;
and wherein R⁵⁸, R⁵⁹ are independently hydrogen or C₁₋₄ alkyl, optionally substituted with -OH.

2. A compound according to claim 1, wherein R¹ is hydrogen.

3. A compound according to claim 1 or 2, wherein R² is hydrogen, chloro, -CH₃, -CH₂F, -CHF₂ or -CN.

4. A compound according to any one of the preceding claims, wherein R³ is hydrogen.

5. A compound according to any one of the preceding claims, wherein A¹ is phenyl or heterocycle containing up to 4 heteroatoms, which are the same or different and selected from the group consisting of -O-, -S-, -S(O)-, -S(O₂)-, -N=, -N(O)= and -N(R⁴)-, wherein R⁴ has the meaning as indicated in claim 1.

6. A compound according to claim 5, wherein A¹ is selected from the group consisting of phenyl, pyridine, pyridine-N oxide and piperidine.

7. A compound according to any one of the preceding claims, wherein Y is a bond or -O-.

8. A compound according to any one of the preceding claims, wherein Z is -C(R⁶⁰R⁶¹)-C(R⁶²R⁶³)-, wherein
R⁶⁰, R⁶¹, R⁶², R⁶³ are independently hydrogen, fluoro, methyl, cyclopropyl or
R⁶⁰ and R⁶¹ form a cyclopropyl ring or
R⁶² and R⁶³ form a cyclopropyl ring or
R⁶⁰ and R⁶² form a cyclopropyl or cyclobutyl ring.

9. A compound according to claim 7, wherein R⁶⁰, R⁶¹, R⁶², R⁶³ are independently hydrogen or fluoro.

10. A compound according to any one of the preceding claims, wherein X is =N-.

11. A compound according to any of the preceding claims, wherein G is -CH(R⁶⁴)-C(R⁶⁵R⁶⁶)-; wherein R⁶⁴, R⁶⁵, R⁶⁶ are independently hydrogen, methyl, -CH₂F, -CHF₂, CF₃ or cyclopropyl or R⁶⁵, R⁶⁶ form together cyclopropyl.

12. A compound according to any one of the preceding claims, wherein D is -CH₂- or -CH(CH₃)- or -C(CH₃)₂- or D¹-D², where D¹ and D² are independently -CH₂-, - CH(CH₃)- or -C(CH₃)₂-.

13. A compound according to any one of the preceding claims, wherein -E is selected from the group consisting of 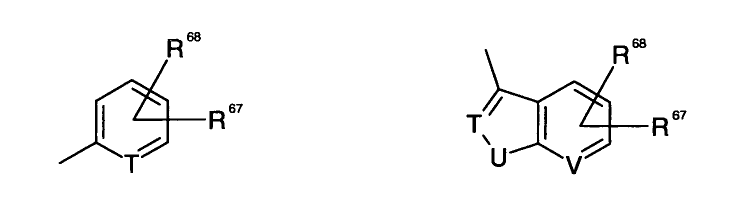 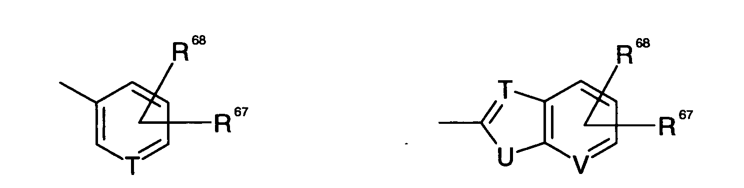 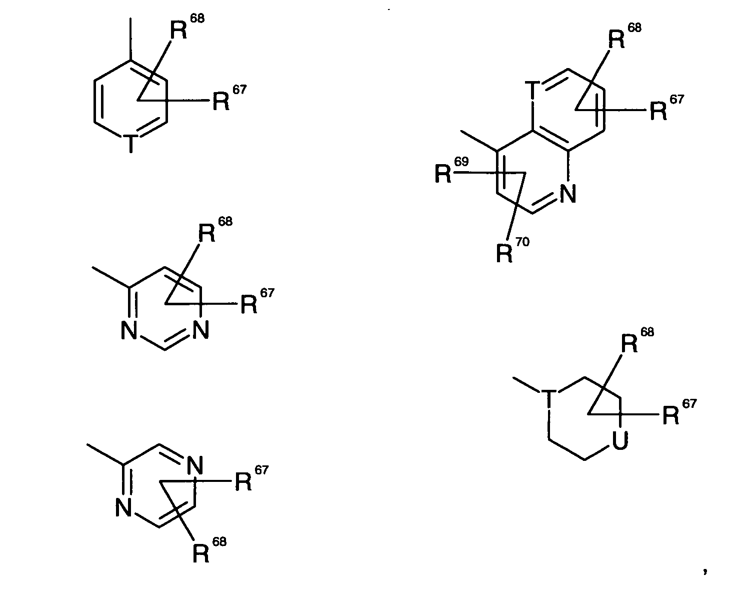 wherein
T and V are independently =CH-, =CR⁷¹- or =N-;
U is -NH-, -NR⁷²-, -O-, or -S-, wherein
R⁶⁷, R⁶⁸, R⁶⁹, R⁷⁰, R⁷¹ are independently selected from the group consisting of
hydrogen;
C₃₋₆ cycloalkyl;
E⁶;
E⁷;
halogen;
-N(R⁷³R⁷⁴);
-OH; and
-COOR⁷⁵ or -C(O)NR⁷⁶R⁷⁷;
and wherein R⁷², R⁷³, R⁷⁴, R⁷⁵, R⁷⁶, R⁷⁷ are independently
hydrogen;
C₁₋₄ alkyl; or
-C(O)-C₁₋₄ alkyl;
E⁶ is selected from the group consisting of C₁₋₆ alkyl; -O-C₁₋₆ alkyl; and -N(R⁷⁸)-C₁₋₆ alkyl, wherein the C₁₋₆ alkyl group is optionally substituted with one or more of
halogen;
-N(R⁷⁹R⁸⁰);
phenyl, optionally substituted with chloro;
heterocycle containing up to 4 heteroatoms, which are the same or different and selected from the group consisting of -O-, -S-, -S(O)-, -S(O₂)-, -N=, -N(O)= and -N(R⁸¹)-, optionally substituted with chloro;
and/or E⁶ is optionally interrupted by one or more of oxygen;
and wherein R⁷⁸, R⁷⁹, R⁸⁰, R⁸¹ are independently hydrogen, C₁₋₄alkyl;
E⁷ is selected from the group consisting of E⁸; -O-E⁸; -N(R⁸²)-E⁸; and -C(O)-E⁸, wherein E⁸ is phenyl or heterocycle containing up to 4 heteroatoms, which are the same or different and selected from the group consisting of -O-, -S-, -S(O)-, -S(O₂)-, -N=, -N(O)= and -N(R⁸³)-; and wherein E⁸ is optionally substituted with chloro or -N(R⁸⁴R⁸⁵);
and wherein R⁸², R⁸³, R⁸⁴, R⁸⁵ are independently hydrogen or C₁₋₄ alkyl.

14. A compound according to claim 13, wherein R⁶⁷, R⁶⁸, R⁶⁹, R⁷⁰, R⁷¹ are independently hydrogen, fluoro, chloro, cyano, phenyl, chlorophenyl, methyl, amino, monomethyl amino, dimethyl amino, pyrrolyl, diazolyl, triazolyl or tertrazolyl.

15. A compound according to claim 1 selected from the group consisting of:

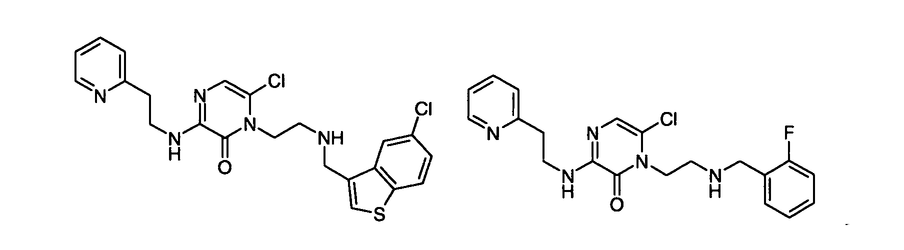

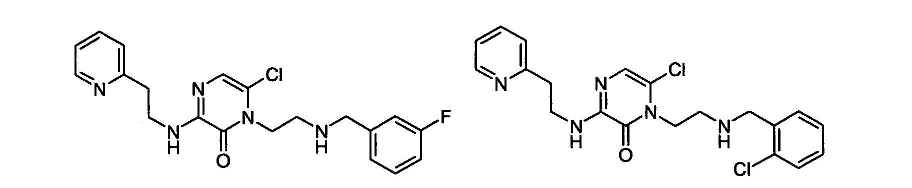

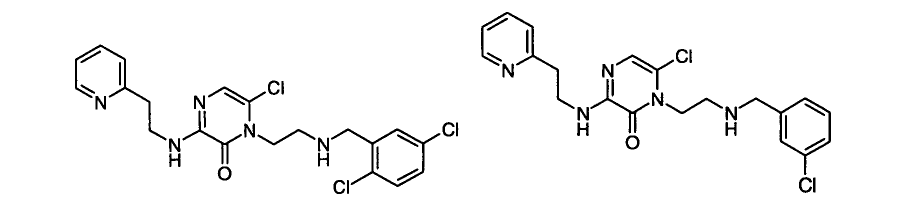

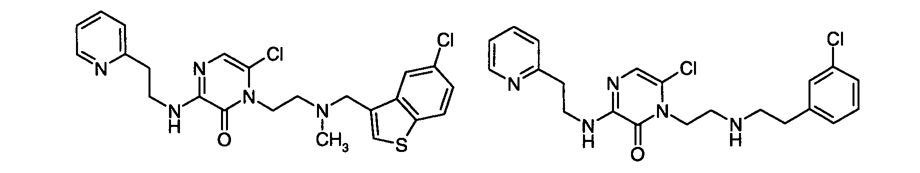

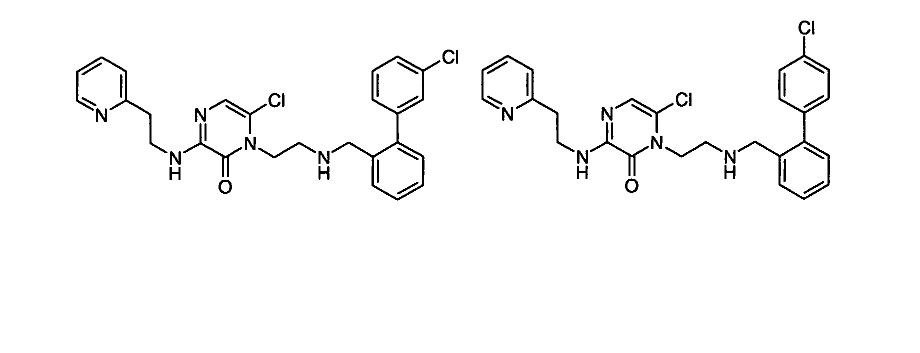

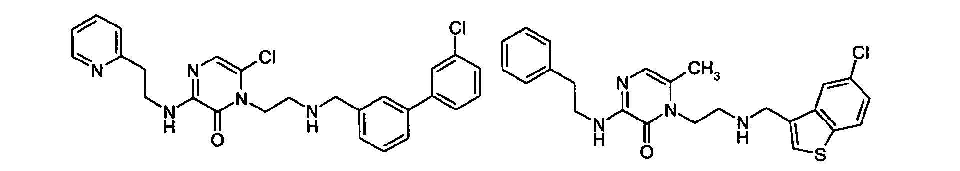

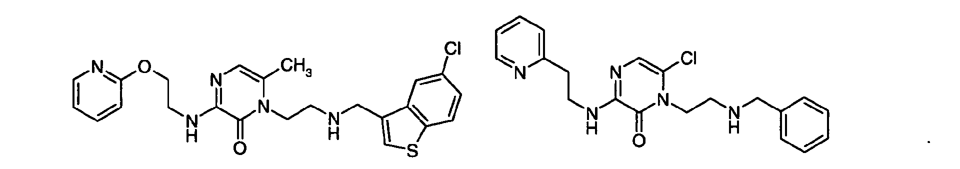

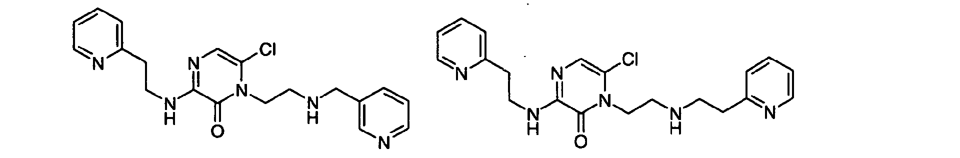

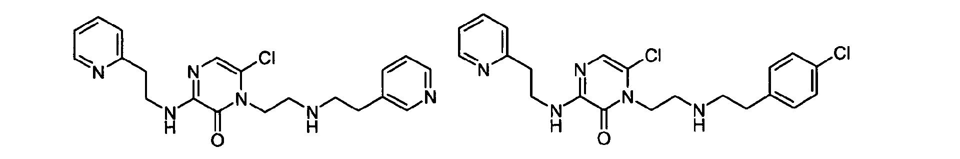

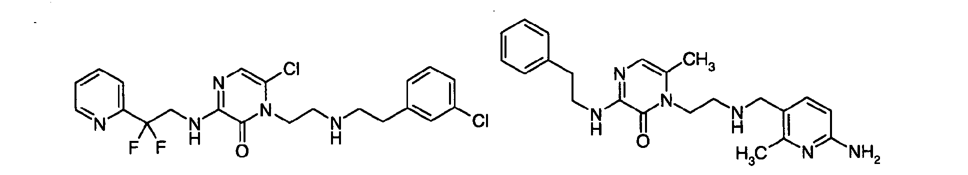

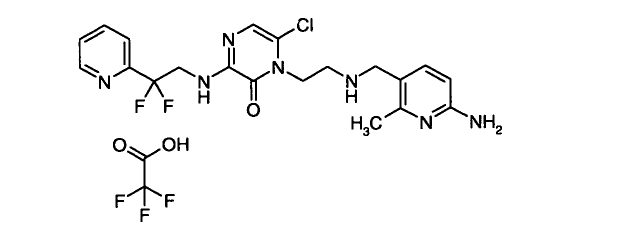

16. A prodrug of a compound according to any one of the claims 1 to 15.

17. A pharmaceutical composition comprising a compound or a mixture of compounds or a pharmaceutically acceptable salt thereof according to any one of the claims 1 to 15 together with a pharmaceutically acceptable carrier.

18. A pharmaceutical composition comprising a prodrug according to claim 16 or a mixture of prodrugs or prodrugs and compounds according to any one of the claims 1 to 15 or a pharmaceutically acceptable salt together with a pharmaceutically acceptable carrier.

19. A pharmaceutical composition according to claim 17 or 18, additionally comprising one or more known anticoagulants.

20. A compound or a pharmaceutically acceptable salt of any one of the claims 1 to 15 for use as a medicament.

21. A prodrug or a pharmaceutically acceptable salt of claim 16 for use as a medicament.

22. Use of a compound or a pharmaceutically acceptable salt of any of the claims 1 to 15 for the manufacture of a medicament for the treatment or prophylaxis of thromboembolism, thrombosis, artherosclerosis, unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels.

23. Use of a prodrug or a pharmaceutically acceptable salt of claim 16 for the manufacture of a medicament for the treatment or prophylaxis of thromboembolism, thrombosis, artherosclerosis, unstable angina, refractory angina, myocardial infarction, transient ischemic attacks, atrial fibrillation, thrombotic stroke, embolic stroke, deep vein thrombosis, disseminated intravascular coagulation, ocular build up of fibrin, and reocclusion or restenosis of recanalized vessels.

24. Use of a compound according to any one of the claims 1 to 15 or a prodrug according to claim 16 as an anticoagulant or thrombin inhibitor.
